# EUROPÄISCHE PATENTSCHRIFT

(11) **EP 1 129 077 B1**
(45) Veröffentlichungstag und Bekanntmachung des Hinweises auf die Patenterteilung: **20.04.2005**
(21) Anmeldenummer: 99963289.6
(22) Anmeldetag: 10.11.1999
(51) Int. Cl.: C07D 221/16, A61K 31/435

(54) **FLUORIERTE 3,4-DIHYDROCHINOLIN-DERIVATIVE ALS NOS-INHIBITOREN**
FLUORINATED 3,4-DIHYDROQUINOLINE DERIVATIVES USED AS NOS INHIBITORS
DERIVES FLUORES DE 3,4-DIHYDROCHINOLINE UTILISES COMME INHIBITEURS DE SYNTHASES DE MONOXYDE D'AZOTE

(30) Priorität: 13.11.1998 DE 19854042
(43) Veröffentlichungstag der Anmeldung: 05.09.2001
(73) Patentinhaber: SCHERING AKTIENGESELLSCHAFT, 13353 Berlin (DE)
(72) Erfinder: JAROCH, Stefan, D-10625 Berlin (DE); REHWINKEL, Hartmut, D-12051 Berlin (DE); HÖLSCHER, Peter, D-10559 Berlin (DE); SÜLZLE, Detlev, D-10589 Berlin (DE); HILLMANN, Margrit, D-13437 Berlin (DE); BURTON, Gerardine, Anne, D-13591 Berlin (DE); MCDONALD, Fiona, Macdougall, D-14055 Berlin (DE)
(74) Vertreter: Pohlman, Sandra M.
(86) Internationale Anmeldenummer: PCT/EP1999/008519
(87) Internationale Veröffentlichungsnummer: WO 2000/029381

(56) Entgegenhaltungen:
- WO-A-96/14844

## Beschreibung

Die Erfindung betrifft fluorierte 3,4-Dihydrochinolin-Derivate, ein Verfahren zu ihrer Herstellung und ihre Verwendung in Arzneimitteln.

In menschlichen Zellen existieren mindestens drei Formen von Stickstoffmonoxid-Synthasen, die Arginin in Stickstoffmonoxid (NO) und Citrullin überführen. Es wurden zwei konstitutive NO-Synthasen (NOS) identifiziert, die als Calzium / Calmodulin abhängige Enzyme im Gehirn (ncNOS oder NOS 1) bzw. im Endothel (ecNOS oder NOS 3) vorhanden sind. Eine weitere Isoform ist die induzierbare NOS (iNOS oder NOS 2), die ein praktisch Ca⁺⁺ unabhängiges Enzym ist und nach Aktivierung unterschiedlicher Zellen durch Endotoxin oder andere Stoffe induziert wird.

NOS-Inhibitoren und insbesondere spezifische Inhibitoren der NOS 1, NOS 2 oder NOS 3 sind daher zur Therapie unterschiedlicher Erkrankungen geeignet, die durch pathologische Konzentrationen von NO in Zellen hervorgerufen oder verschlimmert werden.

Eine Reihe von Reviews informiert über Wirkung und Inhibitoren von NO-Synthasen. Genannt seien beispielsweise: Drugs 1998, **1,** 321 oder Current Pharmac. Design 1997, **3,** 447.

Als NOS-Inhibitoren sind unterschiedliche Verbindungen bekannt. Beispielsweise werden Argininderivate, Aminopyridine, Phenylimidazole und andere beschrieben. Aus WO 96/14844 sind cyclische Amidinderivate als NOS-Inhibitoren bekannt.

Es wurde nun gefunden, daß die erfindungsgemäß substituierten Heterocyclen gegenüber bekannten Verbindungen besonders vorteilhaft als Arzneimittel verwendet werden können.

Die Erfindung betrifft die Verbindungen der Formel I, deren tautomere und isomere Formen oder Salze 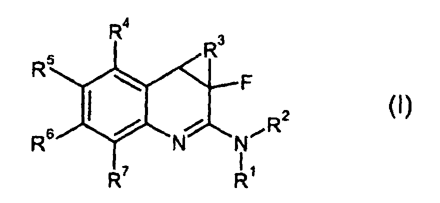 worin
R¹ und R² bedeuten:
Wasserstoff,
R³ bedeutet
einen C₁₋₅-Alkylenrest,
R⁴, R⁵, R⁶ und R⁷ unabhängig voneinander bedeuten
a) Wasserstoff,
b) Halogen,
c) S(O)ₙR⁸,
d) OR⁸,
e) COOR⁸,
f) COR⁸,
g) CONR⁸R¹³,
h) CSNR⁸R¹³,
i) C(NR⁸)NR⁹R¹³,
j) NR¹⁴R¹⁵,
k) C₁₋₆-Alkyl, das gegebenenfalls substituiert ist mit Halogen, OR⁸, SR⁸, NR¹⁴R¹⁵, Phenyl, 5-6-gliedrigem Heteroaryl mit 1-4 N-, S- oder O-Atomen oder C₃₋₇-Cycloalkyl,
l) C₃₋₇-Cycloalkyl,
m) C₂₋₆-Alkenyl, gegebenenfalls substituiert mit Phenyl oder Halogen,
n) C₂₋₆-Alkinyl, gegebenenfalls substituiert mit Phenyl oder Halogen,
o) C₆₋₁₀-Aryl, das gegebenenfalls substituiert ist mit Halogen, CN, C₁₋₄-Alkyl, SR⁸ oder OR⁸,
p) 5 - 6-gliedriges Hetaryl mit 1 bis 4 N-, O- oder S-Atomen, das einen ankondensierten Benzolring enthalten und substituiert sein kann mit Halogen, NO₂, Cyano, -OR⁸, SR⁸, C₁₋₄-Alkyl, CF₃ oder NR⁸R¹³
q) CN,
r) NO₂,
s) CF₃,
t) OCF₃,
R⁴ und R⁵, R⁵ und R⁶ oder R⁶ und R⁷ bilden gemeinsam mit 2 benachbarten Kohlenstoffatomen einen 5- oder 6-gliedrigen Karbocyclus, der mit NR¹⁴R¹⁵ substituiert sein kann,
R⁸, R⁹ und R¹⁰ unabhängig voneinander bedeuten:
a) Wasserstoff,
b) C₁₋₆-Alkyl,
c) C₆₋₁₀-Aryl, das gegebenenfalls substituiert ist mit Halogen oder C₁₋₄-Alkyl
R¹³ bedeutet:
a) Wasserstoff,
b) C₁₋₆-Alkyl, gegebenenfalls substituiert mit Halogen, Amino-, Hydroxyl- oder Sulfhydrylgruppen,
c) C₆₋₁₀-Aryl,
R¹⁴ und R¹⁵ bedeuten unabhängig voneinander:
a) Wasserstoff
b) CO₂R¹⁰
c) C₁₋₆-Alkyl, gegebenfalls substituiert mit Halogen, Hydroxy, C₁₋₄-Alkoxy, Nitro, Amino, C₁₋₆-Alkyl, Trifluormethyl, Carboxyl, Cyano, Carboxamido, C₃₋₇-Cycloalkyl, Indanyl, 1,2,3,4-Tetrahydronaphthyl, C₆₋₁₀-Aryl, 5- oder 6-gliedrigen Heteroaryl mit 1 - 4 Stickstoff-, Sauerstoffoder Schwefelatomen, wobei der Aryl- und der Heteroarylrest mit Halogen, Hydroxy, C₁₋₄-Alkoxy, C₁₋₄-Alkyl, CF₃, NO₂, NH₂, N(C₁₋₄-Alkyl)₂ oder Carboxyl substituiert sein können,
oder
R¹⁴ und R¹⁵ bilden gemeinsam mit dem Stickstoffatom einen 5 - 7-gliedrigen gesättigten Heterocyclus, der ein weiteres Sauerstoff-, Stickstoff- oder Schwefelatom enthalten und mit C₁₋₄-Alkyl, Phenyl, Benzyl oder Benzoyl substituiert sein kann oder einen ungesättigten 5-gliedrigen Heterocyclus, der 1 - 3 N-Atome enthalten und substituiert sein kann mit Phenyl, C₁₋₄ Alkyl Halogen oder CH₂-OH,
und
n bedeutet 0, 1 oder 2.

Die Verbindungen der Formel können als Tautomere, Stereoisomere oder geometrische Isomere vorliegen. Die Erfindung umfaßt auch alle möglichen Isomere wie E- und Z-Isomere, S- und R-Enantiomere, cis- und trans-Diastereomere, Racemate und Gemische derselben einschließlich der tautomeren Verbindungen der Formel Ia und Ib (für R² = Wasserstoff).

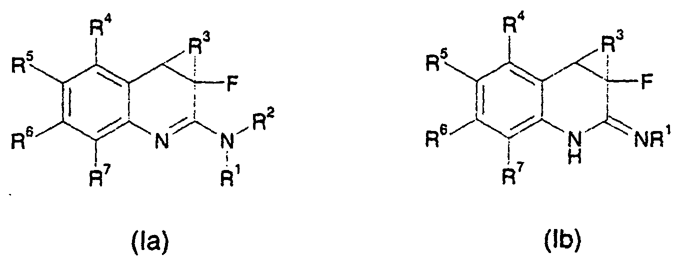

Die physiologisch verträglichen Salze können mit anorganischen und organischen Säuren gebildet werden wie beispielsweise Oxalsäure, Milchsäure, Zitronensäure, Fumarsäure, Essigsäure, Maleinsäure, Weinsäure, Phosphorsäure, Salzsäure, Bromwasserstoffsäure, Schwefelsäure, p-Toluolsulfonsäure, Methansulfonsäure u.a.

Zur Salzbildung von Säuregruppen sind auch die anorganischen oder organischen Basen geeignet, die zur Bildung physiologisch verträglicher Salze bekannt sind wie beispielsweise Alkalihydroxide, wie Natrium- und Kaliumhydroxid, Erdalkalihydroxide wie Calciumhydroxid, Ammoniak, Amine wie Ethanolamin, Diethanolamin, Triethanolamin, N-Methylglucamin, Tris-(hydroxymethyl)-methylamin usw.

Alkyl bedeutet jeweils eine geradkettige oder verzweigte Alkylgruppe wie z.B. Methyl, Ethyl, Propyl, Isopropyl, n-Butyl, sek-Butyl, tert-Butyl, n-Pentyl, sek-Pentyl, tert-Pentyl, Neopentyl, n-Hexyl, sek-Hexyl.

Ist der Alkylrest halogeniert, so kann dieser ein- oder mehrfach halogeniert vorliegen, wobei trifluormethyl bevorzugt ist.

Alkenyl- und Alkinyl-Substituenten sind jeweils geradkettig oder verzweigt. Beispielsweise seien die folgenden Reste genannt: Vinyl, 2-Propenyl, 1-Propenyl, 1-Butenyl, 2-Butenyl, 3-Butenyl, 2-Methyl-2-propenyl, 2-Pentenyl, 4-Hexenyl, Ethinyl, 1-Propinyl, 2-Propinyl, 1-Butinyl, 2-Butinyl.

Unter Cycloalkyl ist jeweils Cyclopropyl, Cyclobutyl, Cyclopentyl, Cyclohexyl oder Cycloheptyl zu verstehen.

Halogen bedeutet jeweils Fluor, Chlor, Brom oder Iod.

Unter Aryl ist jeweils Naphthyl oder insbesondere Phenyl zu verstehen, das einbis dreifach substituiert sein kann. Ebenso können die Phenyl- und Benzylreste ein- bis dreifach gleich oder verschieden substituiert sein.

Der Heteroarylrest kann jeweils einen ankondensierten Benzolring enthalten und ein- bis dreifach gleich oder verschieden substituiert sein und über das Heteroatom oder ein Kohlenstoffatom gebunden sein. Beispielsweise sind die folgenden 5- und 6-Ringheteroaromaten geeignet:

Imidazol, Indol, Isoxazol, Isothiazol, Furan, Oxadiazol, Oxazol, Pyrazin, Pyridazin, Pyrimidin, Pyridin, Pyrazol, Pyrrol, Tetrazol, Thiazol, Triazol, Thiophen, Thiadiazol, Benzimidazol, Benzofuran, Benzoxazol, Isochinolin, Chinolin.

Bevorzugt sind 5- und 6gliedrige Heteroaromaten mit 1 bis 2 Stickstoff-, Sauerstoff- und Schwefelatomen und insbesondere Thienyl und Furanyl wie 2-Furanyl. Als Substituenten der Heteroarylreste sind insbesondere NO₂, CN, Halogen, C₁₋₄-Alkyl und CF₃ geeignet.

Als gesättigte Heterocyclen NR¹⁴R¹⁵ seien beispielsweise Piperidin, Pyrrolidin, Morpholin, Thiomorpholin, Hexahydroazepin und Piperazin genannt. Der Heterocyclus kann 1 - 3fach substituiert sein mit C₁₋₄-Alkyl oder einem gegebenenfalls mit Halogen substituierten Phenyl-, Benzyl- oder Benzoylrest. Beispielsweise seien genannt: N-Methyl-piperazin, 2,6-Dimethylmorpholin, Phenylpiperazin oder 4-(4-Fluorbenzoyl)-piperidin.

Bilden NR¹⁴R¹⁵ gemeinsam mit dem Stickstoffatom einen ungesättigten Heterocyclus, so seien beispielsweise Imidazol, Pyrrol, Pyrazol, Triazol, Benzimidazol und Indazol genannt, die ein- bis zweifach mit Phenyl, C₁₋₄-Alkyl, Halogen, insbesondere Chlor, oder CH₂-OH substituiert sein können.

Bedeutet R¹⁴ oder R¹⁵ Indanyl oder 1,2,3,4-Tetrahydronaphthyl, so kann dieser Rest jeweils in 1- oder 2-Position verknüpft sein.

Bilden R⁴/R⁵, R⁵/R⁶ oder R⁷/R⁸ gemeinsam mit 2 benachbarten Kohlenstoffatomen einen Carbocyclus, so kann dieser in beliebiger Position einoder zweifach mit NR¹⁴R¹⁵ substituiert sein. Bevorzugt ist einfache Substitution. Bevorzugt bedeuten R⁴/R⁵, R⁵/R⁶ oder R⁷/R⁸ C₃₋₄-Alkylen, wobei Substitution R⁵/R⁶ bevorzugt ist.

Der Acylrest leitet sich von geradkettigen oder verzweigten aliphatischen C₁-C₆-Carbonsäuren ab wie beispielsweise Ameisensäure, Essigsäure, Propionsäure, Buttersäure. Trimethylessigsäure oder Capronsäure oder von bekannten Benzolsulfonsäuren, die mit Halogen oder C₁₋₄-Alkyl substituiert sein können, sowie C₁₋₄-Alkansulfonsäuren geeignet wie beispielsweise Methansulfonsäure, p-Toluolsulfonsäure. Bevorzugt seien Alkanoyle genannt.

Als bevorzugte Ausführungsformen von R¹ und R² ist R¹ in der Bedeutung von Wasserstoff zu betrachten und insbesondere bedeuten R¹ und R² Wasserstoff.

R³ bedeutet C₁₋₅-Alkylen. Beispielsweise genannt seien: -(CH₂)₃-, -(CH₂)₄- und -(CH₂)₅-.

Insbesondere sind 1 - 2 Substituenten R⁴, R⁵, R⁶ und R⁷ vorhanden, die nicht Wasserstoff bedeuten.

Bevorzugte Ausführungsformen für R⁴, R⁵, R⁶ und R⁷ sind:
a) Wasserstoff,
b) Halogen,
c) SR⁸
d) OR⁸,
e) COOR⁸,
f) COR⁸,
g) CONR⁸R¹³,
h) NR¹⁴R¹⁵,
i) C₁₋₆-Alkyl, das gegebenenfalls substituiert ist mit Halogen, OR⁸, SR⁸, NR¹⁴R¹⁵, Phenyl, 5 - 6gliedrigem Heteroaryl mit 1 - 4 N-, S- oder O-Atomen oder C₃₋₇-Cycloalkyl,
j) Phenyl, das gegebenenfalls substituiert ist mit Halogen, CN, C₁₋₄-Alkyl, SR⁸ oder OR⁸,
k) 5 - 6gliedriges Heteroaryl mit 1 bis 4 N-, O- oder S-Atomen, das einen ankondensierten Benzolring enthalten und substituiert sein kann mit Halogen, NO₂, Cyano, C₁₋₄-Alkyl, CF₃ oder NR⁸R¹³,
l) CN,
m) NO₂,
n) CF₃,
o) OCF₃ oder
p) R⁴ und R⁵ , R⁵ und R⁶ oder R⁶ und R⁷ bilden gemeinsam mit 2 benachbarten Kohlenstoffatomen eine 5- oder 6gliedrigen Karbocyclus, der mit NR¹⁴R¹⁵ substituiert sein kann.

Besonders bevorzugte Ausführungsformen für R⁴, R⁵, R⁶ und R⁷ sind:
a) Wasserstoff,
b) Halogen,
c) SR⁸,
d) OR⁸,
e) NR¹⁴R¹⁵,
f) C₁₋₆-Alkyl, das gegebenenfalls substituiert ist mit Halogen, OR⁸, NR¹⁴R¹⁵, Phenyl, 5 - 6gliedrigem Heteroaryl mit 1 - 4 N-, S- oder O-Atomen oder C₃₋₇-Cycloalkyl,
g) Phenyl, das gegebenenfalls substituiert ist mit Halogen, CN, C₁₋₄-Alkyl, oder OR⁸,
h) 5 - 6gliedriges Heteroaryl mit 1 bis 4 N-, O- oder S-Atomen, das einen ankondensierten Benzolring enthalten und substituiert sein kann mit Halogen, NO₂, Cyano, C₁₋₄-Alkyl, CF₃ oder NR⁸R¹³,
i) CN,
j) NO₂,
k) CF₃,
l) OCF₃ oder
m) R⁴ und R⁵, R⁶ und R⁷ oder insbesondere R⁵ und R⁶ bilden gemeinsam mit 2 benachbarten Kohlenstoffatomen einen 5- oder 6gliedrigen Karbocyctus, der mit NR¹⁴R¹⁵ substituiert sein kann.

Bevorzugte Ausführungsformen für R¹⁴ und R¹⁵ sind:
a) Wasserstoff,
b) C₁₋₆-Alkyl, gegebenenfalls substituiert mit Halogen, Hydroxy, C₁₋₄-Alkoxy, Nitro, Amino, C₁₋₆-Alkyl, Trifluormethyl, Carboxyl, Cyano, Carboxamido, Phenyl, 5- oder 6gliedrigen Heteroaryl mit 1 - 4 Stickstoff-, Sauerstoff- oder Schwefelatomen, wobei der Phenyl- und der Heteroarylrest mit Halogen, Hydroxy, C₁₋₄-Alkoxy, C₁₋₄-Alkyl, CF₃, NO₂, NH₂, N(C₁₋₄-Alkyl)₂ oder Carboxyl substituiert sein können.

Die Erfindung betrifft auch die Verwendung der erfindungsgemäßen Verbindungen zur Herstellung eines Arzneimittels zur Behandlung von Erkrankungen, die durch die Wirkung von Stickstoffmonoxid in pathologischen Konzentrationen hervorgerufen werden. Dazu zählen neurodegenerative Erkrankungen, inflammatorische Erkrankungen, Autoimmunerkrankungen, Herz-Kreislauf-Erkrankungen.

Beispielsweise seien genannt: Cerebrale Ischaemie, Hypoxie und andere neurodegenerative Erkrankungen, die mit Entzündungen in Verbindung gebracht werden wie Multiple Sklerose, Amyotrophe Lateralsklerose und vergleichbare sklerotische Erkrankungen, Morbus Parkinson, Huntington's Disease, Korsakoff's Disease, Epilepsie, Erbrechen, Streß, Schlafstörungen, Schizophrenie, Depression, Migräne, Schmerz, Hypoglykämie, Demenz wie z.B. Alzheimersche Krankheit, HIV-Demenz und präsenile Demenz.

Ferner eignen sie sich zur Behandlung von Krankheiten des Herz-Kreislauf-Systems und zur Behandlung autoimmuner und/oder inflammatorischer Erkrankungen wie Hypotension, ARDS (adult respiratory distress syndrome), Sepsis oder Septischer Schock, Rheumatoider Arthritis, Osteoarthritis, von insulinabhängiger Diabetes Mellitus (IDDM), entzündlicher Erkrankung des Beckens /Darms (bowel disease), von Meningitis, Glomerulonephritis, akute und chronische Lebererkrankungen, Erkrankungen durch Abstoßung (beispielsweise allogene Herz-, Nieren- oder Lebertransplantationen) oder entzündlichen Hautkrankheiten wie Psoriasis und andere.

Auf Grund ihres Wirkprofils eignen sich die erfindungsgemäßen Verbindungen sehr gut zur Inhibition der neuronalen NOS.

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete Träger-, Hilfsund/oder Zusatzstoffe enthält. Die Applikation kann oral oder sublingual als Feststoff in Form von Kapseln oder Tabletten oder als Flüssigkeit in Form von Lösungen, Suspensionen, Elixieren, Aerosolen oder Emulsionen oder rektal in Form von Suppositorien oder in Form von gegebenenfalls auch subcutan intramuskulär oder intravenös anwendbaren Injektionslösungen oder topisch auch in Form von transdermalen Systemen und Sprays oder intrathekal erfolgen. Als Hilfsstoffe für die gewünschte Arzneimittelformulierung sind die dem Fachmann bekannten inerten organischen und anorganischen Trägermaterialien geeignet wie z.B. Wasser, Gelantine, Gummmi arabicum, Milchzucker, Stärke, Magnesiumstearat, Talk, pflanzliche Öle, Polyalkylenglykole usw. Gegebenenfalls können darüber hinaus Konservierungs-, Stabilisierungs-, Netzmittel, Emulgatoren oder Salze zur Veränderung des osmotischen Druckes oder Puffer enthalten sein.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposome oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 1 - 2000 mg, vorzugsweise 20 - 500 mg, wobei die Dosis als einmal zu verabreichende Einzeldosis oder unterteilt in 2 oder mehreren Tagesdosen gegeben werden kann.

Die NOS-inhibitorische Wirksamkeit der Verbindungen der Formel (I) und deren physiologisch verträglicher Salze kann nach den Methoden von Bredt und Snyder in Proc. Natl. Acad. Sci. USA (1989) 86, 9030-9033 bestimmt werden. Die bNOS-Inhibition der in Beispiel 2 (4-Amino-3a,6-difluor-2,3,3a,9b-tetrahydro-1H-cyclopenta[c]chinolin) beschriebenen Verbindung beträgt IC₅₀ = 590 nM.

Die Herstellung der erfindungsgemäßen Verbindungen erfolgt dadurch, daß man eine Verbindung der Formel (II) oder deren Salz 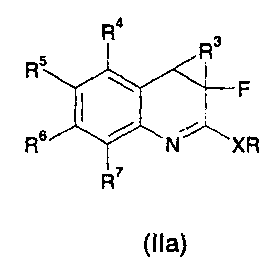 oder 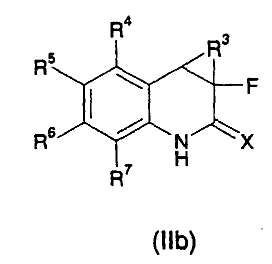 worin R³ bis R⁷ die obige Bedeutung haben, R Methyl oder Ethyl und X Sauerstoff oder Schwefel ist, mit Ammoniak, primären oder sekundären Aminen, Hydroxylamin und seinen Derivaten oder Hydrazin und seinen Derivaten umsetzt und gewünschtenfalls anschließend die Isomeren trennt oder die Salze bildet.

Die Umsetzung mit Ammoniak gelingt unter Druck in Autoklaven bei Ammoniaküberschuß bei tiefen Temperaturen (-78 °C) oder durch Rühren in mit Ammoniak gesättigten Methanol. Bevorzugt werden Thiolactame umgesetzt. Wird mit Aminen umgesetzt, so stellt man aus dem Lactam oder Thiolactam zunächst den Iminoether oder Iminothioether als Zwischenverbindung dar (z.B. mit Methyliodid oder Dimethylsulfat) und setzt diesen mit oder ohne Isolierung mit den entsprechenden Aminen oder deren Salzen um.

Die Isomerengemische können nach üblichen Methoden wie beispielsweise Kristallisation, Chromatographie oder Salzbildung in die Enantiomeren bzw. E/Z-Isomeren aufgetrennt werden. Die Enantiomeren können auch durch Chromatographie an chiralen Phasen sowie durch stereoselektive Synthese erhalten werden.

Die Herstellung der Salze erfolgt in üblicher Weise, indem man eine Lösung der Verbindung der Formel (I) mit der äquivalenten Menge oder einem Überschuß einer Säure, die gegebenenfalls in Lösung ist, versetzt und den Niederschlag abtrennt oder in üblicher Weise die Lösung aufarbeitet.

Soweit die Herstellung der Ausgangsverbindungen nicht beschrieben wird, sind diese bekannt und käuflich oder analog zu bekannten Verbindungen oder nach hier beschriebenen Verfahren herstellbar.

An den Vorstufen werden gewünschtenfalls Sulfide oxidiert, Ester verseift, Säuren verestert, Hydroxygruppen verethert oder acyliert, Amine acyliert, alkyliert, diazotiert, halogeniert, NO₂ eingeführt oder reduziert, mit Isocyanaten oder Isothiocyanaten umgesetzt, die Isomeren getrennt oder die Salze gebildet.

Die Verseifung einer Estergruppe kann basisch oder sauer erfolgen, indem man bei Raumtemperatur oder erhöhter Temperatur bis zur Siedetemperatur des Reaktionsgemisches in Gegenwart von Alkalihydroxiden in Ethanol oder anderen Alkoholen oder mittels Säuren wie z.B. Salzsäure hydrolysiert und ggf. Salze der 3,4-Cycloalkanodihydrochinoline weiterverarbeitet.

Die Veresterung der Carbonsäure geschieht in an sich bekannter Weise mit Diazomethan oder dem entsprechenden Alkohol in Säure oder in Gegenwart eines aktivierten Säurederivats. Als aktivierte Säurederivate kommen zum Beispiel Säurechlorid, -imidazolid oder -anhydrid in Frage.

Zusätzlich kann durch elektrophile aromatische Substitution eine Nitrogruppe oder Halogen, insbesondere Brom, eingeführt werden. Dabei entstehende Gemische können in üblicher Weise, auch mittels HPLC, getrennt werden. Wenn ein Nitril vorliegt, kann dieses nach bekannten Verfahren verseift werden oder in das entsprechende Amin, Tetrazol oder Amidoxim übergeführt werden.

Die Friedel-Crafts-Acylierung wird bei Lactamen vom Typ (IIb, X = O) erfolgreich angewandt; anschließend kann selektiv das Lactam in das Thiolactam übergeführt werden.

Die Einführung einer NO₂-Gruppe gelingt durch eine Reihe von bekannten Nitrierungsmethoden. Beispielsweise kann mit Nitraten oder mit Nitroniumtetrafluoroborat in inerten Lösungsmitteln wie halogenierten Kohlenwasserstoffen oder in Sulfolan oder Eisessig nitriert werden. Möglich ist auch die Einführung z.B. durch Nitriersäure in Wasser, Essigsäure oder konz. Schwefelsäure als Lösungsmittel bei Temperaturen zwischen -10 °C und 30 °C.

Die Reduktion der Nitrogruppe oder ggf. der Cyanogruppe zur Aminogruppe erfolgt katalytisch in polaren Lösungsmitteln bei Raumtemperatur oder erhöhter Temperatur unter Wasserstoffdruck. Als Katalysatoren sind Metalle wie Raney-Nickel oder Edelmetallkatalysatoren wie Palladium oder Platin gegebenenfalls in Gegenwart von Bariumsulfat oder auf Trägem geeignet. Statt Wasserstoff kann auch Ammoniumformiat oder Ameisensäure in bekannter Weise benutzt werden. Reduktionsmittel wie Zinn(II)chlorid oder Titan(III)chlorid können ebenso verwendet werden wie komplexe Metallhydride, eventuell in Gegenwart von Schwermetallsalzen. Für Nitrogruppen bewährt hat sich die Reduktion mit Zink in Wasser-Ethanol-THF/ Ammoniumchlorid oder Eisen in Essigsäure.

Wird eine einfache oder mehrfache Alkylierung einer Aminogruppe oder einer CH-aciden Kohlenstoffposition gewünscht, so kann nach üblichen Methoden beispielsweise mit Alkylhalogeniden alkyliert werden. Gegebenenfalls ist Schutz der Lactamgruppe als Anion durch ein 2. Equivalent Base oder durch eine passende Schutzgruppe erforderlich.

Die Acylierung der Aminogruppe erfolgt in üblicher Weise beispielsweise mit einem Säurehalogenid oder Säureanhydrid gegebenenfalls in Gegenwart einer Base.

Die Einführung der Halogene Chlor, Brom oder Jod über die Aminogruppe kann beispielsweise auch nach Sandmeyer erfolgen, indem man die mit Nitriten intermediär gebildete Diazoniumsalze mit Cu(I)chlorid oder Cu(I)bromid in Gegenwart der entsprechenden Säure wie Salzsäure oder Bromwasserstoffsäure umsetzt oder mit Kaliumjodid umsetzt.

Thiolactame der Formel (IIb, X = S) erhält man beispielsweise aus Lactamen mit Phosphorpentasulfid (P₄S₁₀) oder 2,4-Bis(4-methoxyphenyl)-1,3,2,4-dithiaphosphetan-2,4-disulfid (Lawessons Reagenz) in geeigneten Lösungsmitteln. Verbindungen der Formell (IIa) können beispielsweise durch Umsetzung mit Meerwein-Reagenz (Trimethyloxoniumtetrafluoroborat) erhalten werden.

Die Erfindung betrifft auch die Verbindungen der Formel II b 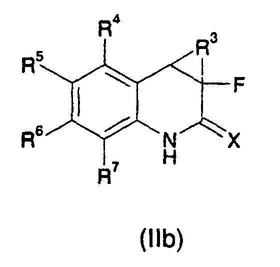 worin R³ bis R⁷ die obige Bedeutung haben und X Sauerstoff oder Schwefel ist, die Zwischenverbindungen bei der Herstellung pharmakologisch wirksamer Verbindungen darstellen und nach den beschriebenen Verfahren erhalten und weiterverarbeitet werden.

Die Herstellung der Verbindungen der Formel (IIb, X = O) erfolgt beispielsweise dadurch, daß man eine Verbindung der Formel (III), 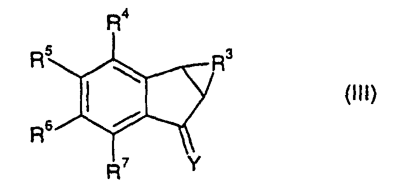 worin R³ bis R⁷ die obige Bedeutung haben und Y = O ist, nach Überführung in das Oxim (Y = NOH), beispielsweise mit einem Hydroxylammoniumsalz und Natriumacetat, einer Beckmann-Umlagerung (R. E. Gawley, *Org. Reactions* **1988,** *35*, 1), beispielsweise in Polyphosphorsäure (vgl. K. Hino, Y. Nagai, H. Uno, *Chem. Pharm. Bull*. **1988,** *36*, 2386) unterwirft und anschließend gegebenenfalls nach Einführung einer Schutzgruppe fluoriert.

Ein anderer Syntheseweg geht von einer Verbindung der Formel (IV) aus, 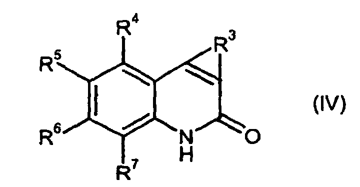 die mit einem Alkali- oder Erdalkalimetal oder einem Amalgam derselben in Alkohol zum Lactam reduziert wird (vgl. B.K. Blount, W.H. Perkin, S.G.P. Plant, *J. Chem. Soc.* **1929,** 1975; R. Brettle, S.M. Shibib, *J.Chem. Soc. Perkin Trans 1*, **1981,** 2912).

Die anschließende Fluorierung zu einer Verbindung der Formel II kann mit N-Fluor-(phenylsulfonyl)imid über das Alkalimetall-Enolat in Lösungsmitteln wie cyclischen Ethern vorgenommen werden. Vorteilhafterweise wird vor der Fluorierung eine Schutzgruppe wie tert. Butoxycarbonyl eingeführt, die nach der Fluorierung in üblicher Weise abgespalten wird.

Die Herstellung der Indanone vom Typ (III) erfolgen in der dem Fachmann bekannten Weise, z. B. nach W. Baker, P.G. Jenes, *J. Chem. Soc.* **1951,** 787; S. Ohta, M. Yamashita, K. Arita, T: Kajiura, J. Kawasaki, K. Noda, M. Izumi, *Chem. Pharm. Bull*. **1995,** *43*, 1294; C. Santelli-Rouvier, M. Santelli, *Synthesis* **1983,**429.

Die Herstellung der Chinolone vom Typ (IV) erfolgt in der dem Fachmann bekannten Weise, z. B. nach B.K. Blount, W.H. Perkin, S.G.P. Plant, *J*. *Chem. Soc.* **1929,** 1975; W. Ried, W. Käppeler, *Liebigs Ann. Chem.* **1965,** *688*, 177; L.A. White, R.C. Storr, *Tetrahedron* **1996,** *52*, 3117.

Die Herstellung der Verbindung der Formel (III) kann beispielsweise dadurch erfolgen, daß man einen Aromaten (V) mit einem aktivierten Säurederivat, wie beispielsweise einem Säurechlorid (Z = CI) oder Anhydrid (Z = OCOR) in Gegenwart einer Lewissäure, wie beispielsweise AlCl₃, SnCl₄, ZnCl₂, SbCl₅, FeCl₃, BF₃-Etherat, in einem inerten Lösungsmittel, wie beispielsweise Dichlormethan, Dichlorethan oder Benzol, bei 0°C bis zur Siedetemperatur des entsprechenden Lösungsmittels umsetzt (s. z.B. W. Baker, P.G. Jones, *J. Chem. Soc.* **1951,** 787). Alternativ können die nach dem Fachmann bekannten Wegen hergestellten Ketone der Formel (VII) (z.B. nach S. Ohta, M. Yamashita, K. Arita, T: Kajiura, I. Kawasaki, K. Noda, M. Izumi, *Chem. Pharm. Bull.* **1995,** *43*, 1294) mit Brönstedtsäuren, wie beispielsweise Schwefelsäure, p-Toluolsulfonsäure, Methansulfonsäure, Phosphorsäure oder Polyphosphorsäure (vgl. C. Santelli-Rouvier, M. Santelli, *Synthesis* **1983**, 429), cyclisiert werden.

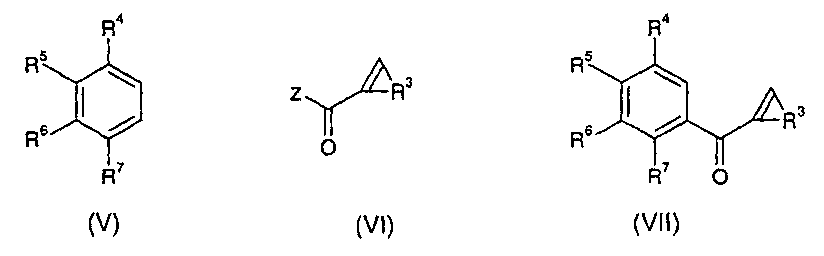

Die Herstellung der Verbindung der Formel (IV) kann beispielsweise dadurch erfolgen, daß ein beta-Ketoamid der Formel (VIII) oder ein Derivat desselben mit einer Säure, beispielsweise Schwefelsäure, Phosphorsäure, Polyphosphorsäure oder Trifluoressigsäure oder Methansulfonsäure, behandelt wird (z.B. B.K. Blount, W.H. Perkin, S.G.P. Plant, *J. Chem. Soc.* **1929,** 1975; W. Ried, W. Käppeler, *Liebigs Ann. Chem.* **1965,** *688,* 177).

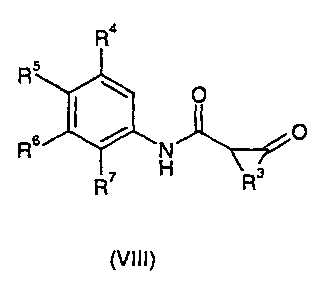

Die Einführung der Substituenten R⁴-R⁷ kann auch auf der Stufe der Verbindungen (III) und (IV) erfolgen und findet wie oben beschrieben statt.

Beispielsweise kann die Herstellung der Verbindungen der Formel II mit R⁴, R⁵, R⁶ oder R⁷ in der Bedeutung eines mit NR¹⁴R¹⁵ substituierten Alkylrestes durch reduktive Aminierung des entsprechenden Aldehyds bzw. wenn R⁴ und R⁵ einen 5- oder 6gliedrigen Carbocyclus bilden, der mit NR¹⁴R¹⁵ substituiert ist, durch reduktive Aminierung des entsprechenden Ketons erfolgen. Wird die Einführung eines Heteroarylrestes NR¹⁴R¹⁵ gewünscht, so kann das entsprechende Halogenderivat nucleophil substituiert werden. Ist eine primäre oder sekundäre Aminogruppe vorhanden, so kann es vorteilhaft sein, diese intermediär zu schützen, beispielsweise durch Einführung einer *tert*-Butoxycarbonylgruppe, die nach der Amidin-Bildung in üblicher Weise abgespalten wird.

Neue Verbindungen wurden durch eine oder mehrere der folgenden Methoden charakterisiert: Schmelzpunkt, Massenspektroskopie, Infrarotspektroskopie, Nuklear-magnetische Resonanzspektroskopie (NMR). NMR Spektren wurden mit einem Bruker 300 MHz Gerät gemessen, die (deuterierten) Lösemittel werden jeweils angegeben und wie folgt abgekürzt: CDCl₃ (Chloroform), CD₃OD ([D₄]-Methanol), DMSO ([D₆]-Dimethylsulfoxid). Verschiebungen sind in delta und ppm angegeben. Es bedeuten: m (Muitiplett, mehrere Signale), s (Singulett), d (Dublett), dd (Doppeldublett usw.), t (Triplett), q (Quartett), H (Wasserstoffprotonen). Ferner bedeuten: THF (Tetrahydrofuran), DMF (N,N-Dimethylformamid), MeOH (Methanol), mL (Milliliter). Alle Lösemittel sind p.A.Qualität, wenn nicht anders vermerkt. Alle Reaktionen werden unter Schutzgas ausgeführt,-es-sei denn es handelt sich um wäßrige Lösungen. Schmelzpunkte werden in Grad Celsius angegeben und sind nicht korrigiert.

Nachfolgend wird die Darstellung von Vorstufen, Zwischenprodukten und Produkten exemplarisch beschrieben.

### Ausgangsverbindungen

### A) 1,2,3,3a,5,9b-Hexahydrocyclopenta[c]chinolin-4-on

### nach Methode A:

2,3,3a,8a-Tetrahydro-1H-cyclopent[a]inden-8-on (1.06 g, 6.0 mmol) (W. Baker, P.G. Jones, *J. Chem. Soc.* **1951,** 787) werden mit Hydroxylammoniumsulfat (1.96 g, 12.0 mmol) und Natriumacetat (24.0 mmol, 3.28 g) in THF-Ethanol-Wasser 1:1:1 (120 ml) gelöst und 5 Tage bei Raumtemperatur gerührt. Das Reaktionsgemisch wird eingeengt und mit Essigester (150 ml) verdünnt, mit ges. NaCl (50 ml) gewaschen, getrocknet (Na₂SO₄) und i. Vak. eingeengt. Der Rückstand wird säulenchromatographisch (SiO₂) mit dem Eluens Hexan-Essigester gereinigt. Ausbeute: 0.88 g (78 %), Schmp. 118 - 20°C; das derart erhaltene Oxim (0.65 g, 3.5 mmol) wird in 120°C heißer Polyphosphorsäure (10 ml) gegeben. Der Ansatz wird 30 min bei 120°C gerührt. Nach dem Abkühlen wird er in Wasser aufgenommen (150 ml) und die wäßrige Lösung mit Essigester extrahiert (3x 150 ml). Die vereinigten Essigester-Extrakte werden getrocknet (Na₂SO₄) und i. Vak. eingeengt. Der Rückstand wird säulenchromatographisch (SiO₂) mit dem Eluens Hexan-Essigester gereinigt. Ausbeute: 202 mg (31 %), Schmp. 133 - 5°C

### nach Methode B:

1,2,3,5-Tetrahydrocyclopenta[c]chinolin-4-on (410 mg, 2.21 mmol) (W. Ried, W. Käppeler, *Liebigs Ann. Chem.* **1965,** *688*, 177) wird in Methanol (25 ml) gelöst und mit Magnesium (538 mg, 22.1 mmol) versetzt. Nach 3 h Rühren bei Raumtemperatur wird der Ansatz filtriert, der Filterrückstand mit Essigester gewaschen, und die vereinigten Filtrate eingeengt. Säulenchromatographische (SiO₂) Reinigung mit dem Eluens Hexan-Essigester liefern 290 mg (71 %) des Produkts.
¹H-NMR (CDCl₃): 1.60-1.80 (m, 3H), 2.03-2.20 (m, 2H), 2.26-2.40 (m, 1H), 2.96 (td, 1H), 3.20-3.32 (m, 1H), 6.78 (dd, 1H), 7.00 (td, 1H), 7.17 (td, 1H), 7.21 (dd, 1H), 8.32 (br. s, 1H).
MS (El) m/e = 187 (M⁺)

### B) 5-tert-Butoxycarbonyl-3a-fluor-1,2,3,3a,5,9b-hexahydrocyclopenta-[c]chinolin-4-on

Eine Lösung von 374 mg (2.0 mmol) 1,2,3,3a,5,9b-Hexahydrocyclopenta-[c]chinolin-4-on in 50 ml THF wird bei Raumtemp. mit 655 mg (3.0 mmol) Pyrokohlensäure-di-*tert*-butylester und 367 mg (3.0 mmol) 4-(Dimethylamino)-pyridin versetzt. Nach 64 h bei Raumtemp. wird das Reaktionsgemisch eingeengt und der Rückstand säulenchromatographisch an Kieselgel mit Hexan-Essigester gereinigt. Dabei werden 350 mg 5-*tert*-Butoxycarbonyl-1,2,3,3a,5,9bhexahydrocyclopenta[c]chinolin-4-on isoliert:
¹H-NMR (CDCl₃): 1.43 - 1.83 (m, 3H), 1.63 (s, 9H), 1.95 - 2.12 (m, 2H), 2.44 (m, 1H), 2.98 (td, 1H), 3.20 (q, 1H), 6.82 (dd, 1H), 7.06 (td, 1H), 7.20 (td, 1H), 7.24 (dd, 1H).
Zu einer Lösung aus 0.25 ml (2.4 mmol) Diethylamin in 20 ml THF werden bei -70 °C 1.5 ml (2.4 mmol) n-Butyllithium (1.6M in Hexan) getropft. Nach ¾ h bei -70 °C wird ein Lösung von 350 mg (1,2 mmol) 5-*tert*-Butoxycarbonyl-1,2,3,3a,5,9b-hexahydrocyclopenta[c]chinolin-4-on in 10 ml THF dazugegeben. Der Ansatz wird 1 h bei -70 °C gerührt, anschließend mit 1.13 g (3.6 mmol) *N*-Fluor-(phenylsulfonyl)imid versetzt und innerhalb von 3 h auf Raumtemp. erwärmt. Nach 2 d Rühren bei Raumtemp. wird das Reaktionsgemisch eingeengt und mit Hexan-Essigester an Kieselgel säulenchromatographisch gereinigt: 360 mg Produkt.
¹H-NMR (CDCl₃): 1.62 (s, 9H), 1.86-2.29 (m, 5H), 2.41 (m, 1H), 3.62 (dm, 1H), 6.84 (d, 1H), 7.12 (t, 1H), 7.21 (d, 1H), 7.27 (t, 1H).

### C) 3a-Fluor-1,2,3,3a,5,9b-hexahydrocyclopenta[c]chinolin-4-on

Eine Lösung von 350 mg (1.1 mmol) 5-*tert*-Butoxycarbonyl-3a-fluor-1,2,3,3a,5,9b-hexahydrocyclopenta[c]chinolin-4-on in 5 mL Dichlormethan werden mit 5 ml Trifluoressigsäure bei Raumtemp. versetzt. Nach 1 h wird der Ansatz mit Wasser (100 ml) und Essigester (100 ml) verdünnt. Die organische Phase wird abgetrennt, mit Wasser (50 ml), ges. NaHCO₃ (2 x 50 ml) und ges. NaCl (50 ml) gewaschen, getrocknet (Na₂SO₄) und i. Vak. eingeengt. Säulenchromatographie des Rückstands an Kieselgel mit Hexan-Essigester liefert 160 mg Produkt.
¹H-NMR (CDCl₃): 1.72 (m, 1H), 1.90 - 2.55 (m, 5H), 3.64 (dt, 1H), 6.89 (d, 1H), 7.08 (t, 1H), 7.20 (d, 1H), 7.24 (t, 1H), 8.93 (br.s, NH).
MS (El) m/e = 205 (M⁺)

### D) 3a-Fluor-1,2,3,3a,5,9b-hexahydrocyclopenta[c]chinolin-4-thion

Eine Lösung von 150 mg (0.73 mmol) 3a-Fluor-1,2,3,3a,5,9b-hexahydrocyclopenta[c]chinolin-4-on in 50 ml THF wird mit 607 mg (1.5 mmol) Lawessons Reagenz versetzt. Nach 1 h Rühren bei Raumtemperatur wird der Ansatz 2 h unter Rückfluß erhitzt. Anschließend wird das Reaktionsgemisch eingeengt und der Rückstand säulenchromatographisch an Kieselgel gereinigt (Eluens: Hexan-Essigester): 150 mg Produkt.
¹H-NMR (CDCl₃): 1.69 (m, 1H), 1.88 - 2.62 (m, 5H), 3.56 (dd, 1H), 6.89 (dd, 1H), 7.16 (td, 1H), 7.22 (d, 1H), 7.27 (t, 1H), 9.79 (br. s, NH).
MS (EI) m/e = 221 (M⁺)

### Beispiel 1

### 4-Amino-3a-fluor-2,3,3a,9b-tetrahydro-1H-cyclopenta[c]chinolin

142 mg (0.64 mmol) 3a-Fluor-1,2,3,3a,5,9b-hexahydrocyclopenta[c]chinolin-4-thion werden in 50 ml 7 M methanolischer Ammoniaklösung gelöst. Nach 1 h Rühren bei Raumtemperatur wird der Ansatz i. Vak. eingeengt und der Rückstand säulenchromatographisch an Kieselgel mit Dichlormethan-Ethanol als Eluens gereinigt: 108 mg Produkt, Schmp. 174 - 6°C.
¹H-NMR (CDCl₃): 1.68 (m, 1H), 1.77 - 2.01 (m, 2H), 2.09 - 2.51 (m, 3H), 3.53 (dd, 1H), 4.34 (br., 2H), 7.00 (t, 1H), 7.04 (d, 1H), 7.16 (t, 1H), 7.19 (d, 1H).
MS (EI) m/e = 204 (M⁺)

### Beispiel 2

### 4-Amino-3a,6-difluor-2,3,3a,9b-tetrahydro-1H-cyclopenta[c]chinolin

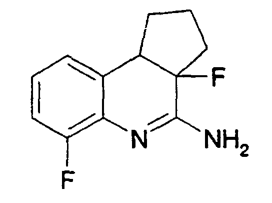

### 5-tert-Butoxycarbonyl-3a,6-difluor-1,2,3,3a,5,9b-hexahydrocyclopenta[c]chinolin-4-on

Eine Lösung von 180 mg (0.9 mmol) 6-Fluor-1,2,3,3a,5,9b-hexahydrocyclopenta[c]chinolin-4-on (WO99/41240) in 10 ml THF wird bei Raumtemp. mit 284 mg (1.3 mmol) Pyrokohlensäure-di-*tert*-butylester und 158 mg (3.0 mmol) 4-(Dimethylamino)pyridin versetzt. Nach 48 h bei Raumtemp. wird das Reaktionsgemisch eingeengt und der Rückstand säulenchromatographisch an Kieselgel mit Hexan-Essigester gereinigt. Dabei werden 240 mg (0.8 mmol) 5-*tert*-Butoxycarbonyl-6-fluor-1,2,3,3a,5,9b-hexahydrocyclopenta[c]chinolin-4-on isoliert, die in 20 ml THF gelöst werden. Dazu wird bei -70 °C 3.2 mL (1.6 mmol) einer 0.5 M Kaliumhexamethyldisilazid-Toluol-Lösung getropft. Der Ansatz wird 1 h bei -70 °C gerührt, anschließend mit 742 mg (2.4 mmol) *N*-Fluor-(phenylsulfonyl)imid versetzt und 4 h bei -70 °C und 20 h bei Raumtemp. gerührt. Das Reaktionsgemisch wird mit Essigester verdünnt, mit Wasser gewaschen, getrocknet (Na₂SO₄) und i. Vak. eingeengt.
Säulenchromatographie an Kieselgel mit Hexan-Essigester liefert 200 mg Produkt.
¹H-NMR (CDCl₃): 1.55 - 1.70 (m, 1H), 1.62.(s, 9H), 1.90 - 2.50 (m, 5H), 3.66 (dm, 1H), 7.00 - 7.18 (m, 3H).

### 3a,6-Difluor-1,2,3,3a,5,9b-hexahydrocyclopenta[c]chinolin-4-on

Eine Lösung von 200 mg (0.6 mmol) 5-*tert-*Butoxycarbonyl-3a,6-difluor-1,2,3,3a,5,9b-hexahydrocyclopenta[c]chinolin-4-on in 2 ml Dichlormethan werden mit 2 ml Trifluoressigsäure bei Raumtemp. versetzt. Nach 30 min wird der Ansatz mit Wasser und Essigester verdünnt. Die organische Phase wird abgetrennt, mit Wasser, ges. NaHCO₃ und ges. NaCl gewaschen, getrocknet (Na₂SO₄) und i. Vak. eingeengt. Säulenchromatographie des Rückstands an Kieselgel mit Hexan-Essigester liefert 60 mg Produkt.
¹H-NMR (CDCl₃): 1.72 (m, 1H), 1.91 - 2.57 (m, 5H), 3.68 (dt, 1H), 6.93 - 7.08 (m, 3H), 7.63 (br.s, NH).

### 3a,6-Difluor-1,2,3,3a,5,9b-hexahydrocyclopenta[c]chinolin-4-thion

Eine Lösung von 60 mg (0.27 mmol) 3a,6-Difluor-1,2,3,3a,5,9b-hexahydrocyclopenta[c]chinolin-4-on in 15 ml THF wird mit 288 mg (0.71 mmol) Lawessons Reagenz versetzt und 1.5 h unter Rückfluß erhitzt. Anschließend wird das Reaktionsgemisch eingeengt und der Rückstand säulenchromatographisch an Kieselgel gereinigt (Eluens: Hexan-Essigester): 50 mg Produkt.
¹H-NMR (CDCl₃): 1.71 (m, 1H), 1.918 - 2.62 (m, 5H), 3.56 (dm, 1H), 6.98 - 7.15 (m, 3H), 9.39 (br. s, NH).

### 4-Amino-3a,6-difluor-2,3,3a,9b-tetrahydro-1H-cyclopenta[c]chinolin

50 mg (0.21 mmol) 3a-Fluor-1,2,3,3a,5,9b-hexahydrocyclopenta[c]chinolin-4-thion werden in 15 ml 7 M methanolischer Ammoniaklösung gelöst. Nach 1 h Rühren bei Raumtemperatur wird der Ansatz i. Vak. eingeengt und der Rückstand säulenchromatographisch an Kieselgel mit Dichlormethan-Ethanol als Eluens gereinigt: 25 mg Produkt.
¹H-NMR (CDCl₃): 1.69 (m, 1H), 1.92 (m, 2H), 2.20 (m, 1H), 2.33 (m, 1H), 2.46 (m, 1H), 3.52 (dd, 1H), 5.86 (br., 2H), 6.88 - 7.02 (m, 3H). MS (EI) m/e = 222 (M⁺)

### Beispiel 3

### 4-Amino-8-brom-3a-fluor-2,3,3a,9b-tetrahydro-1H-cyclopenta[c]chinolin

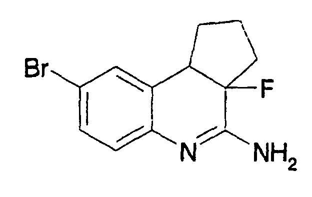

### 5-tert-Butyloxycarbonyl-8-brom-3a-fluor-1,2,3,3a,5,9b-hexahydrocyclopenta[c]chinolin-4-on

Eine Lösung von 1.53 g (5.75 mmol) 8-Brom-1,2,3,3a,5,9b-hexahydrocyclopenta[c]chinolin-4-on (Wo 99/41240) in 120 ml THF wird bei Raumtemp. mit 1.88 g (8.63 mmol) Pyrokohlensäure-di-*tert*-butylester und 1.05 g (8.63 mmol) 4-(Dimethylamino)pyridin versetzt. Nach 5 Tagen bei Raumtemp. wird das Reaktionsgemisch eingeengt und der Rückstand säulenchromatographisch an Kieselgel mit Hexan-Essigester gereinigt. Dabei werden 1.83 g 5-*tert*-Butoxycarbonyl-8-brom-1,2,3,3a,5,9b-hexahydrocyclopenta[c]chinolin-4-on isoliert:
MS (El) m/e = 365/367 (M⁺)
Zu einer Lösung aus 1.56 ml (15.0 mmol) Diethylamin in 100 ml THF werden bei -70 °C 9.4 ml (15.0 mmol) *n*-Butyllithium (1.6M in Hexan) getropft. Nach 1 h bei -70 °C wird eine Lösung von 1.83 g (5.0 mmol) 5-*tert*-Butoxycarbonyl-8-brom-1,2,3,3a,5,9b-hexahydrocyclopenta[c]chinolin-4-on in 20 ml THF dazugegeben. Der Ansatz wird 1 h bei -70 °C gerührt, anschließend mit 6.30 g (20.0 mmol) *N*-Fluor(phenylsulfonyl)imid versetzt und innerhalb von 1 h auf Raumtemp. erwärmt. Nach 15 h Rühren bei Raumtemp. wird das Reaktionsgemisch eingeengt und mit Hexan-Essigester an Kieselgel säulenchromatographisch gereinigt: 1.38 g Produkt.
MS (El) m/e = 383/385 (M⁺)

### 8-Brom-3a-fluor-1,2,3,3a,5,9b-hexahydrocyclopenta[c]chinolin-4-on

Eine Lösung von 1.38 g (3.6 mmol) 5-*tert*-Butoxycarbonyl-8-brom-3a-fluor-1,2,3,3a,5,9b-hexahydrocyclopenta[c]chinolin-4-on in 7 ml Dichlormethan wird mit 7 ml Trifluoressigsäure bei Raumtemp. versetzt. Nach 2 h wird der Ansatz mit Wasser und Essigester verdünnt. Die organische Phase wird abgetrennt, mit Wasser, ges. NaHCO₃ und ges. NaCl gewaschen, getrocknet (Na₂SO₄) und i. Vak. eingeengt. Säulenchromatographie des Rückstands an Kieselgel mit Hexan-Essigester liefert 0.74 g Produkt.
¹H-NMR (CDCl₃): 1.73 (m, 1H), 1.90 - 2.54 (m, 5H), 3.62 (dt, 1H), 6.81 (d, 1H), 7.32 (d, 1H), 7.38 (s, 1H), 9.33 (br.s, NH).
MS (EI) m/e = 283/285 (M⁺)

### 8-Brom-3a-fluor-1,2,3,3a,5,9b-hexahydrocyclopenta[c]chinolin-4-thion

Eine Lösung von 0.74 g (2.6 mmol) 8-Brom-3a-fluor-1,2,3,3a,5,9b-hexahydrocyclopenta[c]chinolin-4-on in 100 ml THF wird mit 2.16 g (5.34 mmol) Lawessons Reagenz 1.5 h unter Rückfluß erhitzt. Anschließend wird das Reaktionsgemisch eingeengt und der Rückstand säulenchromatographisch an Kieselgel mit Hexan-Essigester gereinigt: 0.59 g Produkt.
¹H-NMR (CDCl₃): 1.70 (m, 1H), 1.91 - 2.61 (m, 5H), 3.54 (dm, 1H), 6.81 (d, 1H), 7.37 (d, 1H), 7.43 (s, 1H), 9.91 (br. s, NH).
MS (EI) m/e = 299/301 (M⁺).

### 4-Amino-8-brom-3a-fluor-2,3,3a,9b-tetrahydro-1H-cyclopenta[c]chinolin

75 mg (0.25 mmol) 8-Brom-3a-fluor-1,2,3,3a,5,9b-hexahydrocyclopenta[c]-chinolin-4-thion werden in 20 ml 7 M methanolischer Ammoniaklösung gelöst. Nach 1.5 h Rühren bei Raumtemperatur wird der Ansatz i. Vak. eingeengt und der Rückstand säulenchromatographisch an Kieselgel mit Essigester gereinigt: 60 mg Produkt, Schmp. 220°C.
¹H-NMR (CDCl₃): 1.61- 2.00 (m, 3H), 2.10 - 2.52 (m, 3H), 3.50 (dd, 1H), 5.40 (br., 2H), 6.92 (d, 1H), 7.27 (m, 2H).
MS (EI) m/e = 282/284 (M⁺).

### Beispiel 4

### 4-Amino-8-chlor-3a-fluor-2,3,3a,9b-tetrahydro-1H-cyclopenta[c]chinolin

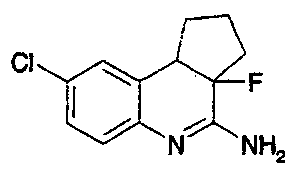

### 5-tert-Butyloxycarbonyl-8-chlor-3a-fluor-1,2,3,3a,5,9b-hexahydrocyclopenta[c]chinolin-4-on

Eine Lösung von 1.70 g (7.66 mmol) 8-Chlor-1,2,3,3a,5,9b-hexahydrocyclopenta[c]chinolin-4-on (WO 99/41240) in 120 ml THF wird bei Raumtemp. mit 2.50 g (11.5 mmol) Pyrokohlensäure-di-*tert*-butylester und 1.40 g (11.5 mmol) 4-(Dimethylamino)pyridin versetzt. Nach 22 h bei Raumtemp. wird das Reaktionsgemisch eingeengt und der Rückstand säulenchromatographisch an Kieselgel mit Hexan-Essigester gereinigt. Dabei werden 2.35 g 5-*tert*-Butoxycarbonyl-8-chlor-1,2,3,3a,5,9b-hexahydrocyclopenta[c]chinolin-4-on isoliert:
¹H-NMR (CDCl₃): 1.59 - 1.86 (m, 3H), 1.63 (s, 9H), 2.07 (m, 2H), 2.45 (m, 1H), 2.99 (td, 1H), 3.20 (q, 1H), 6.78 (d, 1H), 7.19 (dd, 1H), 7.24(dd, 1H).

Zu einer Lösung aus 2.24 ml (21.5 mmol) Diethylamin in 100 ml THF werden bei -70 °C 13.4 ml (21.5 mmol) *n*-Butyllithium (1.6M in Hexan) getropft. Nach 1 h bei -70 °C wird eine Lösung von 2.30 g (7.2 mmol) 5-*tert*-Butoxycarbonyl-8-chlor-1,2,3,3a,5,9b-hexahydrocyclopenta[c]chinolin-4-on in 50 ml THF dazugegeben. Der Ansatz wird 1 h bei -70 °C gerührt, anschließend mit 9.02 g (28.6 mmol) *N*-Fluor(phenylsulfonyl)imid versetzt und innerhalb von 1 h auf Raumtemp. erwärmt. Nach 15 h Rühren bei Raumtemp. wird das Reaktionsgemisch eingeengt und mit Hexan-Essigester an Kieselgel säulenchromatographisch gereinigt: 2.20 g Produkt.
¹H-NMR (CDCl₃): 1.58 - 1.74 (m, 1H), 1.65 (s, 9H), 1.90 - 2.50 (m, 5H), 3.60 (dm, 1H), 6.82 (d, 1H), 7.20 (dd, 1H), 7.26 (d, 1H).

### 8-Chlor-3a-fluor-1,2,3,3a,5,9b-hexahydrocyclopenta[c]chinolin-4-on

Eine Lösung von 2.20 g (6.5 mmol) 5-*tert*-Butoxycarbonyl-8-chlor-3a-fluor-1,2,3,3a,5,9b-hexahydrocyclopenta[c]chinolin-4-on in 10 ml Dichlormethan wird mit 10 ml Trifluoressigsäure bei Raumtemp. versetzt. Nach 2 h wird der Ansatz mit Wasser und Essigester verdünnt. Die organische Phase wird abgetrennt; mit Wasser, ges. NaHCO₃ und ges. NaCl gewaschen, getrocknet (Na₂SO₄) und i. Vak. eingeengt. Säulenchroamtographie des Rückstands an Kieselgel mit Hexan-Essigester liefert 0.83 g Produkt.
¹H-NMR (CDCl₃, [D]₆-DMSO): 1.48 (m, 1H), 1.62 - 2.26 (m, 5H), 3.32 (dt, 1H), 6.66 (d, 1H), 6.87 (d, 1H), 6.94 (s, 1H), 10.10 (br.s, NH).
MS (El) m/e = 239 (M⁺).

### 8-Chlor-3a-fluor-1,2,3,3a,5,9b-hexahydrocyclopenta[c]chinolin-4-thion

Eine Lösung von 0.83 g (3.5 mmol) 8-Chlor-3a-fluor-1,2,3,3a,5,9b-hexahydrocyclopenta[c]chinolin-4-on in 100 ml THF wird mit 2.86 g (7.1 mmol) Lawessons Reagenz 1.5 h unter Rückfluß erhitzt. Anschließend wird das Reaktionsgemisch eingeengt und der Rückstand säulenchromatographisch an Kieselgel mit Hexan-Essigester gereinigt: 0.86 g Produkt.
MS (El) m/e = 255 (M⁺).

### 4-Amino-8-chlor-3a-fluor-2,3,3a,9b-tetrahydro-1H-cyclopenta[c]chinolin

90 mg (0.35 mmol) 8-Chlor-3a-fluor-1,2,3,3a,5,9b-hexahydrocyclopenta[c]-chinolin-4-thion werden in 20 ml 7 M methanolischer Ammoniaklösung gelöst. Nach 2 h Rühren bei Raumtemperatur wird der Ansatz i. Vak. eingeengt und der Rückstand säulenchromatographisch an Kieselgel mit Essigester gereinigt: 73 mg Produkt, Schmp. 223 °C.
¹H-NMR (CDCl₃, [D]₆-DMSO): 1.39 (m, 1H), 1.60 (m, 2H), 1.83 - 2.02 (m, 2H), 2.15 (m, 1H), 3.17 (dd, 1H), 6.65 (d, 1H), 6.77 (dd, 1H), 6.83 (br. s, 1H).
MS (El) m/e = 238 (M⁺)

### Beispiel 5

### 4-Amino-7-methylaminomethyl-3a-fluor-2,3,3a,5,9b-tetrahydro-1Hcyclopenta[c]chinolin Dihydrochlorid

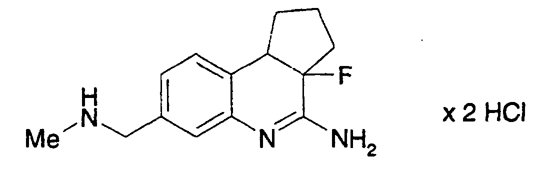

### 5-tert-Butyloxycarbonyl-3a-fluor-7-(2-furanyl)-1,2,3,3a,5,9b-hexahydrocyclopenta[c]chinolin-4-on

Eine Lösung von 5.07 g (20.0 mmol) 7-(2-Furanyl)-1,2,3,3a,5,9b-hexahydrocyclopenta[c]chinolin-4-on (WO 99/41240) in 350 ml THF wird bei Raumtemp. mit 6.55 g (30.0 mmol) Pyrokohlensäure-di-*tert*-butylester und 3.67 g (30.0 mmol) 4-(Dimethylamino)pyridin versetzt. Nach 7 h bei Raumtemp. wird das Reaktionsgemisch eingeengt und der Rückstand säulenchromatographisch an Kieselgel mit Hexan-Essigester gereinigt. Dabei werden 2.53 g 5-*tert*-Butoxycarbonyl-7-(2-furanyl)-1,2,3,3a,5,9b-hexahydrocyclopenta[c]chinolin-4-on isoliert:
¹H-NMR (CDCl₃): 1.65 - 1.80 (m, 3H), 1.65 (s, 9H), 2.08 (m, 2H), 2.43 (m, 1H), 3.00 (td, 1H), 3.22 (q, 1H), 6.48 (dd, 1H), 6.60 (d, 1H), 7.14 (d, 1H), 7.23 (d, 1H), 7.36 (dd, 1H), 7.46 (d, 1H).
Zu einer Lösung von 1.72 ml (16.5 mmol) Diethylamin in 100 ml THF werden bei -70 °C 10.3 ml (16.5 mmol) *n*-Butyllithium (1.6 M in Hexan) getropft. Nach 1 h bei -70 °C wird eine Lösung von 1.94 g (5.5 mmol) 5-*tert*-Butoxycarbonyl-7-(2-furanyl)-1,2,3,3a,5,9b-hexahydrocyclopenta[c]chinolin-4-on in 30 ml THF dazugegeben. Der Ansatz wird 1 h bei -70 °C gerührt, anschließend mit 6.94 g (22.0 mmol) *N*-Fluor(phenylsulfonyl)imid versetzt und innerhalb von 1 h auf Raumtemp. erwärmt. Nach 15 h Rühren bei Raumtemp. wird das Reaktionsgemisch eingeengt und mit Hexan-Essigester an Kieselgel säulenchromatographisch gereinigt: 1.44 g Produkt.
¹H-NMR (CDCl₃): 1.65 (m, 1H), 1.68 (s, 9H), 1.87 - 2.52 (m, 5H), 3.65 (dm, 1H), 6.47 (dd, 1H), 6.63 (d, 1H), 7.17 (d, 1H), 7.28 (d, 1H), 7.43 (dd, 1H), 7.47 (d, 1H).

### 3a-Fluor-7-(2-furanyl)-1,2,3,3a,5,9b-hexahydrocyclopenta[c]chinolin-4-on

Eine Lösung von 1.44 g (3.9 mmol) 5-*tert*-Butoxycarbonyl-3a-fluor-7-(2-furanyl)-1,2,3,3a,5,9b-hexahydrocyclopenta[c]chinolin-4-on in 20 ml Dichlormethan wird mit 8 ml Trifluoressigsäure bei Raumtemp. versetzt. Nach 2 h wird der Ansatz mit Wasser und Essigester verdünnt. Die organische Phase wird abgetrennt, mit Wasser, ges. NaHCO₃ und ges. NaCl gewaschen, getrocknet (Na₂SO₄) und i. Vak. eingeengt. Säulenchromatographie des Rückstands an Kieselgel mit Hexan-Essigester liefert 0.53 g Produkt.
Schmp. 203 - 6°C.
¹H-NMR (CDCl₃): 1.73 (m, 1H), 1.91 - 2.55 (m, 5H), 3.65 (dm, 1H), 6.49 (dd, 1H), 6.67 (d, 1H), 7.10 (d, 1H), 7.26 (d, 1H), 7.38 (dd, 1H), 7.48 (d, 1H), 8.23 (br.s, 1H).

### 3a-Fluor-1,2,3,3a,5,9b-hexahydrocyclopenta[c]chinolin-4-on-7-carbonsäure

Eine Suspension von 0.5 g (1.8 mmol) 3a-Fluor-7-(2-furanyl)-1,2,3,3a,5,9bhexahydrocyclopenta[c]chinolin-4-on in 63 ml Acetonitril-Tetrachlorkohlenstoff-Wasser (2:1:2) wird mit 5.9 g (27.6 mmol) Natriumperiodat und 0.05 g (0.4 mmol) Ruthenium(IV)oxid versetzt und 4 h bei Raumtemp. gerührt. Der Ansatz wird mit Wasser verdünnt und mit Essigester extrahiert. Die vereinigte Extrakte werden getrocknet (Na₂SO₄) und i. Vak. eingeengt. Der Rückstand wird in 0.5 M Kalilauge aufgenommen. Diese Lösung wird mit Methyl-*tert*-butylether gewaschen, mit konz. Salzsäure angesäuert und mit Essigester extrahiert. Die vereinigten Extrakte werden getrocknet (Na₂SO₄) und i. Vak. eingeengt: 0.31 g Produkt.
¹H-NMR ([D]₆-DMSO): 1.60 (m, 1H), 1.84 (m, 1H), 1.96 - 2.16 (m, 3H), 2.32 (m, 1H), 3.56 (dt, 1H), 7.43 (d, 1H), 7.53 (s, 1H), 7.58 (dd, 1H), 10.71 (br.s, 1H).

### 7-[{(N-tert-Butyloxycarbonyl)methylamino}methyl]-3a-fluor-1,2,3,3a,5,9b-hexahydrocyclopenta[c]chinolin-4-on

Eine Lösung von 0.31 g (1.24 mmol) 3a-Fluor-1,2,3,3a,5,9b-hexahydrocyclopenta[c]chinolin-4-on-7-carbonsäure in 35 ml THF wird mit 0.20 ml (1.44 mmol) Triethylamin und 0.14 ml (1.44 mmol) Chlorameisensäure-ethylester behandelt. Nach 10 min bei Raumtemp. werden 0.14 g (3.72 mmol) Natriumborhydrid und innerhalb von 15 min 19 ml Methanol dazugegeben. Nach 15 h wird der Ansatz mit Essigester verdünnt, mit 20 % Citronensäure gewaschen, getrocknet (Na₂SO₄) und i. Vak. eingeengt. Säulenchromatographie mit Hexan-Essigester liefert 3a-Fluor-7-hydroxymethyl-1,2,3,3a,5,9b-hexahydrocyclopenta[c]chinolin-4-on, das in 50 ml THF gelöst und bei 0 °C mit je 4 Portione 0.34 ml (2.5 mmol) Triethylamin und 0.19 ml (2.5 mmol) Methansulfonylchlorid versetzt wird. Nach beendeter Reaktion wird der Ansatz auf Eiswasser gegossen und mit Essigester extrahiert. Die vereinigten Extrakte werden getrocknet (Na₂SO₄) und i. Vak. eingeengt. Der Rückstand wird in 50 ml 2 M methanolischer Methylamin-Lösung aufgenommen und 4 h unter Rückfluß erhitzt. Die flüchtigen Bestandteile werden i. Vak. entfernt und der Rückstand in 50 ml Dichlormethan gelöst. 0.81 g (3.72 mmol) Pyrokohlensäure-di-*tert*-butylester werden dazugegeben und nach 3 h weitere 0.40 g (1.86 mmol). Nach 2 h wird der Ansatz mit Dichlormethan verdünnt, mit Wasser gewaschen, getrocknet (Na₂SO₄) und i. Vak. eingeengt.
Säulenchromatographie mit Hexan-Essigester liefert 49 mg Produkt.
¹H-NMR CDCl₃): 1.48 (s, 9H), 1.71 (m, 1H), 1.87 - 2.53 (m, 5H), 2.83 (s, 3H), 3.62 (dm, 1H), 4.36 (s, 2H), 6.68 (s, 1H), 6.93 (d, 1H), 7.20 (d, 1H), 8.23/8.51 (br.s, 1H).

### 7-[{(N-tert-Butyloxycarbonyl)methylamino}methyl]-3a-fluor-1,2,3,3a,5,9bhexahydrocyclopenta[c]chinolin-4-thion

Eine Lösung von 70 mg (0.2 mmol) 7-[{(N-*tert*-Butyloxycarbonyl)methylamino}methyl]-3a-fluor-1,2,3,3a,5,9b-hexahydrocyclopenta[c]chinolin-4-on in 10 ml THF wird mit 160 mg (0.4 mmol) Lawessons Reagenz 1.5 h unter Rückfluß erhitzt. Anschließend wird das Reaktionsgemisch eingeengt und der Rückstand säulenchromatographisch an Kieselgel mit Hexan-Essigester gereinigt: 72 mg Produkt.
¹H-NMR CDCl₃): 1.49 (s, 9H), 1.68 (m, 1H), 1.89 - 2.61 (m, 5H), 2.84 (s, 3H), 3.53 (dm, 1H), 4.39 (s, 2H), 6.70 (s, 1H), 7.02 (d, 1H), 7.24 (d, 1H), 9.47 (br.s, 1H).

### 4-Amino-7-methylaminomethyl-3a-fluor-2,3,3a,5,9b-tetrahydro-1Hcyclopenta[c]chinolin Dihydrochlorid

67 mg (0.18 mmol) 7-[{(N-*tert*-Butyloxycarbonyl)methylamino}methyl]-3a-fluor-1,2,3,3a,5,9b-hexahydrocyclopenta[c]chinolin-4-thion werden in 10 ml 7 M ammoniakalischem Methanol 2 h bei Raumtemp. gerührt. Nach Abdestillieren der flüchtigen Bestandteile i. Vak. wird der Rückstand säulenchromatographisch mit Dichlormethan-Methanol an Kieselgel gereinigt: 52 mg (0.15 mmol) 4-Amino-7-[{(N-*tert*-butyloxycarbonyl)methylamino}methyl]-3a-fluor-2,3,3a,5,9b-tetrahydro-1H-cyclopenta[c]chinolin. Diese werden in 2 ml 4 M salzsaurem Dioxan 1.5 h bei Raumtemp. gerührt. Die flüchtigen Bestandteile werden i. Vak. entfernt.
MS (EI) m/e = 247 ([M - 2 HCl]⁺).

### Beispiel 6

### 4-Amino-7-[2-(methylamino)ethyl]-3a-fluor-2,3,3a,5,9b-tetrahydro-1Hcyclopenta[c]chinolin Dihydrochlorid

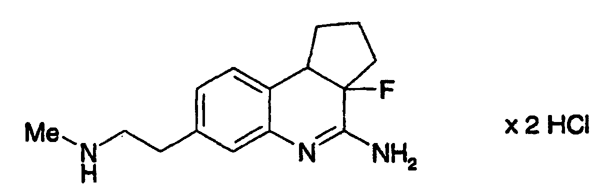

### 7-Vinyl-1,2,3,5-tetrahydrocyclopenta[c]chinolin-4-on

Eine Lösung von 2.0 g (7.6 mmol) 7-Brom-1,2,3,5-tetrahydrocyclopenta[c]-chinolin-4-on, 2.6 ml (9.9 mmol) Vinyltributylzinn und 0.44 g (0.38 mmol) Tetrakis(triphenylphosphin)palladium wird entgast und mit Stickstoff belüftet. Nach 6stündigem Erwärmen auf 110 °C wird der Ansatz eingeengt und der Rückstand auf Kieselgel aufgezogen. Säulenchromatographie an Kieselgel mit Hexan-Essigester liefert 1.41 g Produkt.
¹H-NMR (CDCl₃): 2.26 (pent., 2H), 3.05 (t, 2H), 3.16 (t, 2H), 5.41 (d, 1H), 5.91 (d, 1H), 6.82 (dd, 1H), 7.33 (d, 1H), 7.38 (s, 1H), 7.49 (d, 1H), 11.22 (br.s, 1H).

### 7- Oxiranyl-1,2,3,5-tetrahydrocyclopenta[c]chinolin-4-on

Eine Lösung von 1.41 g (6.7 mmol) 7-Vinyl-1,2,3,5-tetrahydrocyclopenta[c]-chinolin-4-on in 200 ml Chloroform wird bei Raumtemp. mit *m*CPBA versetzt. Nach 15 h bei Raumtemp. wird der Ansatz mit ges. Na₂SO₃ (2 x 100 ml) gewaschen, getrocknet (Na₂SO₄) und i. Vak. eingeengt. Säulenchromatographie an Kieselgel mit Hexan-Essigester liefert 0.59 g Produkt.
¹H-NMR (CDCl₃): 2.25 (pent., 2H), 2.85 (dd, 1H), 3.03 (t, 2H), 3.13 (t, 2H), 3.20 (dd, 1H), 3.98 (dd, 1H), 7.11 (dd, 1H), 7.35 (d, 1H), 7.49 (d, 1H), 11.44 (br.s, 1H).

### 7-(2-Hydroxyethyl)-1,2,3,3a,5,9b-hexahydrocyclopenta[c]chinolin-4-on

Eine Lösung von 0.59 g (2.6 mmol) 7- Oxiranyl-1,2,3,5-tetrahydrocyclopenta[c]-chinolin-4-on in 100 ml Methanol wird mit 1.26 g (52.0 mmol) Magnesium und 0.06 ml Essigsäure versetzt. Der Ansatz wird 4 h bei Raumtemp. gerührt und mit weiteren 0.63 g (26.0 mmol) Magnesium versetzt. Nach 15 h bei Raumtemp. wird das Reaktionsgemisch mit 200 ml 10 % Salzsäure angesäuert und mit Essigester (3 x 200 ml) extrahiert. Die vereinigten Extrakte werden getrocknet (Na₂SO₄) und i. Vak. eingeengt. Säulenchromatographie an Kieselgel mit Hexan-Essigester liefert 0.39 g Produkt.
¹H-NMR (CDCl₃): 1.57 - 1.78 (m, 3H), 2.01 - 2.18 (m, 2H), 2.29 (m, 1H), 2.82 (t, 2H), 2.93 (td, 1H), 3.23 (q, 1H), 3.86 (t, 2H), 6.62 (d, 1H), 6.88 (dd, 1H), 7.15 (d, 1H), 8.31 (br.s, 1H).

### 7-[2-(tert-Butyldimethylsilyloxy)ethyl]-1,2,3,3a,5,9b-hexahydrocyclopenta[c]chinolin-4-on

Bei 0 °C werden 0.62 g (5.4 mmol) *tert*-Butyldimethylsilylchlorid und 0.72 g (10.7 mol) Imidazol zu einer Lösung von 0.62 g (2.7 mmol) 7-(2-Hydroxyethyl)-1,2,3,3a,5,9b-hexahydrocyclopenta[c]chinolin-4-on in 10 ml DMF gegeben. Nach 4 h bei Raumtemp. wird der Ansatz mit Ether verdünnt, mit Wasser, 10 % Citronensäure und ges. NaHCO₃ gewaschen, getrocknet (Na₂SO₄) und i. Vak. eingeengt. Säulenchromatographie mit Hexan-Essigester an Kieselgel liefert 0.79 g Produkt.
¹H-NMR (CDCl₃): 0.02 (s, 6H), 0.90 (s, 9H), 1.71 (m, 3H), 2.12 (m, 2H), 2.32 (m, 1H), 2.78 (t, 2H), 2.96 (td, 1H), 3.26 (q, 1H), 3.80 (t, 2H), 6.60 (d, 1H), 6.86 (dd, 1H), 7.14 (d, 1H), 7.93 (br. s, 1H).

### 5-tert-Butyloxycarbonyl-7-[2-(tert-butyldimethylsilyloxy)ethyl]-3a-fluor-1,2,3,3a,5,9b-hexahydrocyclopenta[c]chinolin-4-on

Eine Lösung von 0.79 g (2.3 mmol) 7-[2-(*tert*-Butyldimethylsilyloxy)ethyl]-1,2,3,3a,5,9b-hexahydrocyclopenta[c]chinolin-4-on in 60 ml THF wird mit 0.75 g (3.4 mmol) Pyrokohlensäure-di-*tert*-butylester und 0.42 g (3.4 mmol) 4-(Dimethylamino)pyridin 18 h bei Raumtemp. gerührt. Weitere 0.37 g (1.7 mmol) Pyrokohlensäure-di-*tert*-butylester und 0.21 g (1.7 mmol) 4-(Dimethylamino)pyridin werden hinzugefügt, und nach 4 h wird das Lösungsmittel i. Vak. entfernt und der Rückstand säulenchromatographisch an Kieselgel mit Hexan-Essigester gereinigt: 1.0 g 5-*tert*-Butyloxycarbonyl-7-[2-(*tert*-butyldimethylsilyloxy)ethyl]-1,2,3,3a,5,9b-hexahydrocyclopenta[c]chinolin-4-on.
¹H-NMR (CDCl₃): 0.01 (s, 6H), 0.87 (s, 9H), 1.65 (s, 9H), 1.72 (m, 3H), 2.04 (m, 2H), 2.41 (m, 1H), 2.79 (t, 2H), 2.96 (td, 1H), 3.19 (q, 1H), 3.78 (t, 2H), 6.64 (d, 1H), 6.93 (dd, 1H), 7.14 (d, 1H).

Zu einer Lösung von 0.71 ml (6.8 mmol) Diethylamin in 50 ml THF werden bei -70 °C 4.3 ml (6.8 mmol) *n*-Butyllithium (1.6 M in Hexan) getropft. Nach 1 h bei -70 °C wird eine Lösung von 1.0 g (2.3 mmol) 5-*tert*-Butyloxycarbonyl-7-[2-(*tert*butyldimethylsilyloxy)ethyl]-1,2,3,3a,5,9b-hexahydrocyclopenta[c]chinolin-4-on in 10 ml THF dazugegeben. Der Ansatz wird 1 h bei -70 °C gerührt, anschließend mit 2.87 g (9.1 mmol) *N*-Fluor(phenylsulfonyl)imid versetzt und innerhalb von 1 h auf Raumtemp. erwärmt. Nach 15 h Rühren bei Raumtemp. wird das Reaktionsgemisch eingeengt und mit Hexan-Essigester an Kieselgel säulenchromatographisch gereinigt: 0.77 g Produkt.
¹H-NMR (CDCl₃): 0.01 (s, 6H), 0.88 (s, 9H), 1.61 - 1.71 (m, 1H), 1.67 (s, 9H), 1.87 - 2.49 (m, 5H), 2.80 (t, 2H), 3.62 (dm, 1H), 3.81 (m, 2H), 6.68 (d, 1H), 7.00 (dd, 1H), 7.21 (d, 1H).

### 3a-Fluor-7-(2-hydroxyethyl)-1,2,3,3a,5,9b-hexahydrocyclopenta[c]chinolin-4-on

0.77 g (1.7 mmol) 5-*tert*-Butyloxycarbonyl-7-[2-(*tert*-butyldimethylsilyloxy)ethyl]-3a-fluor-1,2,3,3a,5,9b-hexahydrocyclopenta[c]chinolin-4-on werden in 10 ml 4 M salzsaurem Dioxan 2 h bei Raumtemp. gerührt. Der Ansatz wird i. Vak. eingeengt, der Rückstand in Essigester aufgenommen, mit ges. NaHCO₃ gewaschen, getrocknet (Na₂SO₄) und i. Vak. eingeengt. Säulenchromatographie an Kieselgel mit Hexan-Essigester liefert 0.26 g Produkt.
MS (El) m/e = 249 (M⁺).

### 7-[2-{(N-tert-Butyloxycarbonyl)methylamino}ethyl]-3a-fluor-1,2,3,3a,5,9bhexahydrocyclopenta[c]chinolin-4-on

Eine Lösung von 0.15 g (0.46 mmol) 3a-Fluor-7-(2-hydroxyethyl)-1,2,3,3a,5,9bhexahydrocyclopenta[c]chinolin-4-on in 20 ml THF wird bei -5 °C mit 0.07 ml (0.5 mmol) Triethylamin und 0.04 ml (0.5 mmol) Methansulfonylchlorid versetzt und 1.5 h bei 0 °C gerührt. Der Ansatz wird mit 10 % Citronensäure angesäuert und mit Essigester extrahiert. Die vereinigten Extrakte werden mit Wasser gewaschen, getrocknet (Na₂SO₄) und i. Vak. eingeengt. Der Rückstand wird in 20 ml 2 M methanolischer Methylamin-Lösung aufgenommen und 4 h unter Rückfluß erhitzt. Die flüchtigen Bestandteile werden i. Vak. entfernt und der Rückstand in 50 ml Dichlormethan gelöst. 0.31 g (1.4 mmol) Pyrokohlensäure-di-*tert*-butylester werden dazugegeben und nach 4 h weitere 0.10 g. Nach 6 h wird der Ansatz mit Dichlormethan verdünnt, mit Wasser gewaschen, getrocknet (Na₂SO₄) und i. Vak. eingeengt. Säulenchromatographie mit Hexan-Essigester liefert 0.12 g Produkt.
¹H-NMR (CDCl₃): 1.40 (s, 9H), 1.68 (m, 2H), 1.88 - 2.52 (m, 4H), 2.76 (t, 2H), 2.83 (s, 3H), 3.44 (t, 2H), 3.60 (dm, 1H), 6.66 (s, 1H), 6.90 (br., 1H), 7.17 (d, 1H), 8.31/8.57 (br. 1H).

### 7-[2-{(N-tert-Butyloxycarbonyl)methylamino}ethyl]-3a-fluor-1,2,3,3a,5,9bhexahydrocyclopenta[c]chinolin-4-thion

Eine Lösung von 0.12 g (0.33 mmol) 7-[2-{(N-*tert*-Butyloxycarbonyl)methylamino}ethyl]-3a-fluor-1,2,3,3a,5,9b-hexahydrocyclopenta[c]chinolin-4-on in 15 ml THF wird mit 0.27 g (0.67 mmol) Lawessons Reagenz 1.5 h unter Rückfluß erhitzt. Anschließend wird das Reaktionsgemisch eingeengt und der Rückstand säulenchromatographisch an Kieselgel mit Hexan-Essigester gereinigt: 0.10 g Produkt.
¹H-NMR (CDCl₃): 1.41 (s, 9H), 1.67 (m, 2H), 1.88 - 2.59 (m, 4H), 2.76 (br.t, 2H), 2.84 (s, 3H), 3.44 (br.t, 2H), 3.50 (dm, 1H), 6.68 (br., 1H), 7.00 (br., 1H), 7.21 (d, 1H), 9.57 (br. 1H).

### 4-Amino-7-[2-{(N-tert-butyloxycarbonyl)methylamino}ethyl]-3a-fluor-2,3,3a,5,9btetrahydro-1H-cyclopenta[c]chinolin

0.10 g (0.26 mmol) 7-[2-{(N-*tert*-Butyloxycarbonyl)methylamino}ethyl]-3a-fluor-1,2,3,3a,5,9b-hexahydrocyclopenta[c]chinolin-4-thion werden in 10 ml 7 M ammoniakalischem Methanol 2 h bei Raumtemp. gerührt. Nach Abdestillieren des flüchtigen Bestandteile i. Vak. wird der Rückstand säulenchromatographisch mit Dichlormethan-Methanol an Kieselgel gereinigt: 89 mg Produkt.
¹H-NMR (CDCl₃): 1.43 (s, 9H), 1.56 - 2.50 (m, 6H), 2.75 (br.t, 2H), 2.83 (s, 3H), 3.42 (br.m, 2H), 3.48 (dd, 1H), 5.31 (s, 2H), 6.82 (br., 1H), 6.90 (s, 1H), 7.10 (d, 1H).
MS (FAB) m/e = 362 (M⁺).

### 4-Amino-7-[2-(methylamino)ethyl]-3a-fluor-2,3,3a,5,9b-tetrahydro-1Hcyclopenta[c]chinolin Dihydrochlorid

85 mg (0.23 mmol) 4-Amino-7-[2-{(N-*tert*-butyloxycarbonyl)methylamino}methyl]-3a-fluor-2,3,3a,5,9b-tetrahydro-1H-cyclopenta[c]chinolin werden in 5 ml 4 M salzsaurem Dioxan 1 h bei Raumtemp. gerührt. Die flüchtigen Bestandteile werden i. Vak. entfernt.
MS (CI) m/e = 262 ([MH - 2 HCI]⁺).

### Beispiel 7

### 4-Amino-7-[3-(3-chlorbenzylamino)propyl]-3a-fluor-2,3,3a,9b-tetrahydro-1Hcyclopenta[c]chinolin Dihydrochlorid

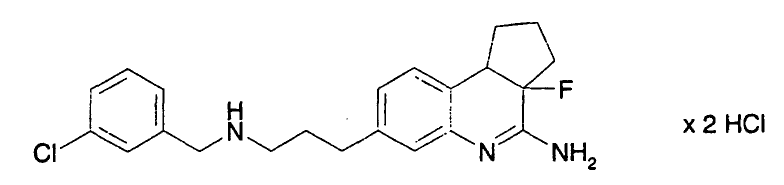

### 7-(2-Methoxycarbonylethenyl)-1,2,3,5-tetrahydrocyclopenta[c]chinolin-4-on

Eine Suspension von 528 mg (2.0 mmol) 7-Brom-1,2,3,5-tetrahydrocyclopenta[c]chinolin-4-on (WO 99/41240), 0.36 ml (4.0 mmol) Acrylsäuremethylester, 116 mg (0.1 mmol) Tetrakis(triphenylphosphin)palladium und 0.56 ml (4.0 mmol) Triethylamin in 25 ml DMF wird 3 h bei 120 °C gerührt. Der Ansatz wird mit Essigester verdünnt, mit Wasser gewaschen, getrocknet (Na₂SO₄) und i. Vak. eingeengt. Reinigung des Rückstands an Kieselgel mit Dichlormethan-Ethanol liefert 550 mg Produkt.
¹H-NMR ([D₆]-DMSO): δ = 2.12 (pent, 2H), 2.80 (t, 2H), 3.12 (t, 2H), 3.77 (s, 3H), 6.61 (d, 1H), 7.52 (s, 1H), 7.56 (s, 2H), 7.67 (d, 1H), 11.19 (br.s, 1H).

### 7-(2-Methoxycarbonylethyl)-1,2,3,3a,5,9b-hexahydrocyclopenta[c]chinolin-4-on

Eine Lösung von 550 mg (2.0 mmol) 7-(Methoxycarbonylethenyl)-1,2,3,5-tetrahydrocyclopenta[c]chinolin-4-on in 130 ml Methanol-THF 3:1 wird mit 972 mg (40.0 mmol) Magnesium versetzt und 24 h bei Raumtemp. gerührt. Das Reaktionsgemisch wird über Glasfaser filtriert, der Filterrückstand mit Dichlormethan-Methanol gewaschen und die vereinigten Filtrate i. Vak. eingeengt. Reinigung des Rückstands an Kieselgel liefert 120 mg Produkt.
¹H-NMR (CDCl₃): δ = 1.57 - 1.77 (m, 3H), 2.02 - 2.18 (m, 2H), 2.31 (m, 1H), 2.63 (t, 2H), 2.91 (t, 2H), 2.94 (td, 1H), 3.23 (q, 1H), 3.69 (s, 3H), 6.58 (d, 1H), 6.84 (dd, 1H), 7.12 (d, 1H), 8.11 (br.s, 1H).

### 7-(3-Hydroxypropyl)-1,2,3,3a,5,9b-hexahydrocyclopenta[c]chinolin-4-on

Eine Lösung von 4.0 g (14.6 mmol) 7-(2-Methoxycarbonylethyl)-1,2,3,3a,5,9bhexahydrocyclopenta[c]chinolin-4-on in 60 ml THF und 100 ml Methanol wird 24 h mit 29 ml 1 M wäßriger Natronlauge gerührt. Mit 10 % Schwefelsäure wird der Ansatz auf pH 5 eingestellt und mit Dichlormethan und Essigester extrahiert. Die vereinigten Extrakte werden getrocknet (Na₂SO₄) und i. Vak. eingeengt. Nach Säulenchromatographie an Kieselgel mit Dichlormethan-Methanol werden 3.4 g 7-(Carboxyethyl)-1,2,3,3a,5,9b-hexahydrocyclopenta[c]chinolin-4-on isoliert.
¹H-NMR (CDCl₃, [D]₆-DMSO): δ = 1.17 - 1.40 (m, 3H), 1.70 (m, 2H), 1.93 (m, 1H), 2.21 (t, 2H), 2.50 (m, 3H), 2.84 (q, 1H), 6.37 (d, 1H), 6.46 (dd, 1H), 6.73 (d, 1H), 9.27 (s, 1H).

3.1 g (11.9 mmol) der Säure werden in 200 ml THF gelöst und bei 0 °C mit 1.23 ml (13.1 mmol) Chlorameisensäure-ethylester und 1.84 ml (13.1 mmol) Triethylamin versetzt. Nach 10 min werden 2.25 g (59.5 mmol) Natriumborhydrid dazugegeben und innerhalb von 10 min 200 ml Methanol zugetropft. Nach 30 min bei Raumtemp. wird der Ansatz i. Vak. konzentiert und der Rückstand mit Essigester-Dichlormethan (9:1) extrahiert. Die Extrakte werden mit ges. NaCl gewaschen, getrocknet (Na₂SO₄) und. i. Vak. eingengt. Säulenchromatographie an Kieselgel mit Hexan-Essigester liefert 2.7 g Produkt.
¹H-NMR (CDCl₃): δ =.1.58 -1.79 (m, 4H), 1.89 (m, 2H); 2.10 (m, 2H), 2.30 (m, 1H), 2.67 (t, 2H), 2.94 (td, 1H), 3.23 (q, 1H), 3.70 (t, 2H), 6.60 (d, 1H), 6.87 (dd, 1H), 7.18 (d, 1H), 8.26 (br.s, 1H).

### 7-[3-(tert-Butyldimethylsilyloxy)propyl]-1,2,3,3a,5,9b-hexahydrocyclopenta[c]-chinolin-4-on

Bei 0 °C werden 3.65 g (24.5 mmol) *tert*-Butyldimethylsilylchlorid und 3.29 g (48.9 mol) Imidazol zu einer Lösung von 3.0 g (12.2 mmol) 7-(3-Hydroxypropyl)-1,2,3,3a,5,9b-hexahydrocyclopenta[c]chinolin-4-on in 50 ml DMF gegeben. Nach 3 h wird der Ansatz mit Essigester verdünnt, mit Wasser, 10 % Citronensäure und ges. NaHCO₃ gewaschen, getrocknet (Na₂SO₄) und i. Vak. eingeengt. Säulenchromatographie mit Hexan-Essigester an Kieselgel liefert 4.62 g Produkt.
¹H-NMR (CDCl₃): δ = 0.05 (s, 6H), 0.91 (s, 9H), 1.54 - 1.84 (m, 3H), 2.00 - 2.14 (m, 2H), 2.28 (m, 1H), 2.61 (t, 2H), 2.92 (td, 1H), 3.21 (q, 1H), 3.62 (t, 2H), 6.53 (d, 1H), 6.82 (dd, 1H), 7.09 (d, 1H), 7.82 (br.s, 1H).

### 5-tert-Butyloxycarbonyl-7-[3-(tert-butyldimethylsilyloxy)propyl]-3a-fluor-1,2,3,3a,5,9b-hexahydrocyclopenta[c]chinolin-4-on

Eine Lösung von 4.62 g (12.9 mmol) 7-[3-(*tert*-Butyldimethylsilyloxy)propyl]-1,2,3,3a,5,9b-hexahydrocyclopenta[c]chinolin-4-on in 250 ml THF wird mit 4.22 g (19.3 mmol) Pyrokohlensäure-di-*tert*-butylester und 2.37 g (19.3 mmol) 4-(Dimethylamino)pyridin 24 h bei Raumtemp. gerührt. Das Lösungsmittel wird i. Vak. entfernt und der Rückstand säulenchromatographisch an Kieselgel mit Hexan-Essigester gereinigt: 5.33 g 5-*tert*-Butyloxycarbonyl-7-[3-(*tert*-butyldimethylsilyloxy)propyl]-1,2,3,3a,5,9b-hexahydrocyclopenta[c]chinolin-4-on.
MS (EI) m/e = 459 (M⁺).
Zu 0.50 g (1.1 mmol) 5-*tert*-Butyloxycarbonyl-7-[3-(*tert*-butyldimethylsilyloxy)propyl]-1,2,3,3a,5,9b-hexahydrocyclopenta[c]chinolin-4-on in 20 ml THF werden bei -70 ° C 4.4 ml (2.2 mmol) 0.5 M Kaliumhexamethyldisilazid-Toluol-Lösung getropft. Nach 1 h bei -70 °C werden 0.95 g (3.3 mmol) N-Fluor-(phenylsulfonyl)imid dazugegeben und 4 h bei -70 °C und 18 h bei Raumtemp. gerührt. Der Ansatz wird mit Essigester verdünnt, mit Wasser gewaschen, getrocknet (Na₂SO₄) und i. Vak. eingeengt. Säulenchromatographie an Kieselgel mit Hexan-Essigester Liefert 0.40 g Produkt.
¹H-NMR (CDCl₃): δ = 0.06 (s, 6H), 0.91 (s, 9H), 1.62 (m, 2H), 1.65 (s, 9H), 1.81 (m, 2H), 1.87 - 2.45 (m, 4H), 2.64 (t, 2H), 3.59 (m, 1H), 3.63 (t, 2H), 6.66 (s, 1H), 6.97 (d, 1H), 7.18 (d, 1H).

### 3a-Fluor-7-(3-hydroxypropyl)-1,2,3,3a,5,9b-hexahydrocyclopenta[c]chinolin-4-on

0.40 g (0.84 mmol) 5-*tert*-Butyloxycarbonyl-7-[3-(*tert*-butyldimethylsilyloxy)-propyl]-3a-fluor-1,2,3,3a,5,9b-hexahydrocyclopenta[c]chinolin-4-on werden in 5 ml Dichlormethan gelöst und mit 5 ml Trifluoressigsäure behandelt. Nach 1 h bei Raumtemp. wird der Ansatz mit Essigester verdünnt, mit Wasser und ges. NaHCO₃ gewaschen, getrocknet (Na₂SO₄) und i. Vak. eingeengt. Säulenchromatographie an Kieselgel mit Hexan-Essigester liefert 60 mg Produkt und 100 mg (0.28 mmol) 3a-Fluor-7-(3-trifluoroxycarbonylpropyl)-1,2,3,3a,5,9bhexahydrocyclopenta[c]chinolin-4-on, die mit 192 mg (1.4 mmol) Kaliumcarbonat in 10 ml MeOH in weitere 60 mg Produkt übergeführt werden.
¹H-NMR (CDCl₃): δ = 1.60 (br., 1H), 1.72 (m, 2H), 1.91 (m, 2H), 1.95 - 2.52 (m, 4H), 2.67 (t, 2H), 3.60 (m, 1H), 3.71 (t, 2H), 6.69 (s, 1H), 6.92 (d, 1H), 7.16 (d, 1H), 8.63 (br.s, 1H).

### 7-[3-(3-Chlorbenzylamino)propyl]-3a-fluor-1,2,3,3a,5,9b-hexahydrocyclopenta[c]chinolin-4-on

Bei -70 °C wird zu 0.08 ml (0.92 mmol) Oxalylchlorid in 10 ml Dichlormethan eine Lösung von 0.10 ml (1.4 mol) DMSO in 5 ml Dichlormethan getropft. Nach 10 min bei -70 °C werden eine Lösung von 120 mg (0.46 mmol) 3a-Fluor-7-(3-hydroxypropyl)-3a-fluor-1,2,3,3a,5,9b-hexahydrocyclopenta[c]chinolin-4-on in 10 ml Dichlormethan und nach 2 h bei -70 °C 0.57 ml (4.1 mmol) Triethylamin hinzugefügt. Der Ansatz wird auf Raumtemp. erwärmt, noch 1 h gerührt und i. Vak. eingeengt. Der Rückstand wird in 20 ml 1,2-Dichlorethan und 10 ml THF gelöst, mit 0.06 ml (0.69 mmol) 3-Chlorbenzylamin, 145 mg (0.69 mmol) Natrium(triacetoxy)borhydrid und 0.27 ml (4.6 mmol) Essigsäure versetzt und 24 h bei Raumtemp. gerührt. Der Ansatz wird mit Essigester verdünnt, mit Wasser gewaschen, getrocknet (Na₂SO₄) u. i. Vak. eingeengt. Säulenchromatographie an Kieselgel mit Essigester-Methanol liefert 120 mg Produkt.
¹H-NMR (CDCl₃): δ = 1.64 (m, 1H), 1.70 - 2.20 (m, 5H), 2.32 (m, 2H), 2.61 (m, 4H), 3.46 (br., 2H), 3.58 (dt, 1H), 3.80 (s, 2H), 6.80 (s, 1H), 6.90 (d, 1H), 7.24 (d, 1H), 7.33 - 7.43 (m, 3H), 7.48 (s, 1H), 10.53 (br.s, 1H).

### 7-[3-(N-tert-Butoxycarbonyl-3-chlorbenzylamino)propyl]-3a-fluor-1,2,3,3a,5,9bhexahydrocyclopenta[c]chinolin-4-on

Eine Lösung von 120 mg (0.31 mmol) 7-[3-(3-Chlorbenzylamino)propyl]-3a-fluor-1,2,3,3a,5,9b-hexahydrocyclopenta[c]chinolin-4-on in 20 ml Dichlormethan wird mit 81 mg (0.37 mmol) Di-*tert*-butylkohlensäureanhydrid versetzt und 24 h bei Raumtemp. gerührt. Der Ansatz wird mit Dichlormethan verdünnt, mit Wasser gewaschen, getrocknet (Na₂SO₄) und i. Vak. eingeengt. Säulenchromatographie an Kieselgel mit Hexan-Essigester liefert 100 mg Produkt.
¹H-NMR (CDCl₃): δ = 1.49 (s, 9H), 1.62 - 2.57 (m, 10H), 3.22 (br., 2H), 3.59 (dm, 1H), 4.41 (s, 2H), 6.54 (s, 1H), 6.86 (d, 1H), 7.10 (br., 1H), 7.14 (d, 1H), 7.21 (s, 1H), 7.25 (m, 2H), 7.59 (br., 1H).

### 7-[3-(N-tert-Butoxycarbonyl-3-chlorbenzylamino)propyl]-3a-fluor-1,2,3,3a,5,9bhexahydrocyclopenta[c]chinolin-4-thion

Eine Lösung von 100 mg (0.21 mmol) 7-[3-(*N-tert*-Butoxycarbonyl-3-chlorbenzylamino)propyl]-3a-fluor-1,2,3,3a,5,9b-hexahydrocyclopenta[c]chinolin-4-on und 223 mg (0.55 mmol) Lawessons Reagenz in 15 ml THF wird 1.5 h unter Rückfluß erhitzt. Nach Einengen i. Vak. wird der Rückstand säulenchromatograpisch mit Hexan-Essigester gereinigt: 80 mg Produkt.
MS (Cl) m/e = 503 (M⁺).

### 4-Amino-7-[3-(N-tert-butoxycarbonyl-3-chlorbenzylamino)propyl]-3a-fluor-2,3,3a,9b-tetrahydro-1H-cyclopenta[c]chinolin

80 mg (0.16 mmol) 7-[3-(*N-tert*-Butoxycarbonyl-3-chlorbenzylamino)propyl]-3afluor-1,2,3,3a,5,9b-hexahydrocyclopenta[c]chinolin-4-thion werden in 15 ml 7 M ammoniakalischem Methanol 1 h bei Raumtemp. gerührt. Nach Abdestillieren des flüchtigen Bestandteile i. Vak. wird der Rückstand säulenchromatographisch mit Dichlormethan-Methanol an Kieselgel gereinigt: 60 mg Produkt.
MS (Cl) m/e = 486 (M⁺).

### 4-Amino-7-[3-(3-chlorbenzylamino)propyl]-3a-fluor-2,3,3a,9b-tetrahydro-1Hcyclopenta[c]chinolin Dihydrochlorid

60 mg (0.12 mmol) 4-Amino-7-[3-(*N-tert*-butoxycarbonyl-3-chlorbenzylamino)-propyl]-3a-fluor-2,3,3a,9b-tetrahydro-1H-cyclopenta[c]chinolin werden in 5 ml 4 M salzsaurem Dioxan 1 h bei Raumtemp. gerührt. Die flüchtigen Bestandteile werden i. Vak. entfernt: 70 mg.
MS (Cl) m/e = 385 ([M - 2 HCl]⁺).

Analog können hergestellt werden:
4-Amino-3a,7-difluor-2,3,3a,9b-tetrahydro-1H-cyclopenta[c]chinolin
4-Amino-3a,8-difluor-2,3,3a,9b-tetrahydro-1H-cyclopenta[c]chinolin
4-Amino-3a-fluor-7-methoxy-2,3,3a,9b-tetrahydro-1H-cyclopenta[c]chinolin
4-Amino-3a-fluor-8-methoxy-2,3,3a,9b-tetrahydro-1H-cyclopenta[c]chinolin
4-Amino-3a-fluor-7-methyl-2,3,3a,9b-tetrahydro-1H-cyclopenta[c]chinolin
4-Amino-3a-fluor-8-methyl-2,3,3a,9b-tetrahydro-1H-cyclopenta[c]chinolin
4-Amino-3a-fluor-8-nitro-2,3,3a,9b-tetrahydro-1H-cyclopenta[c]chinolin
4-Amino-3a-fluor-8-trifluormethyl-2,3,3a,9b-tetrahydro-1H-cyclopenta[c]chinolin
4-Amino-3a-fluor-8-cyano-2,3,3a,9b-tetrahydro-1H-cyclopenta[c]chinolin
4-Amino-3a-fluor-7-(2-furanyl)-2,3,3a,9b-tetrahydro-1H-cyclopenta[c]chinolin
4-Amino-7-(3-chlorbenrylamino)-3a-fluor-1,2,3,3a,7,8,9,10b-octahydrodicyclopenta[c,g]chinolin Dihydrochlorid

## Patentansprüche

1. Verbindungen der Formel I, deren tautomere und isomere Formen oder Salze 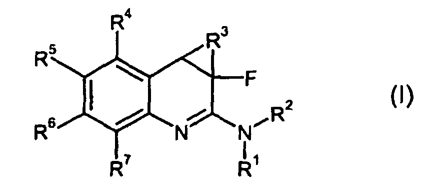 worin
R¹ und R² bedeuten:
Wasserstoff,
R³ bedeutet
einen C₁₋₅-Alkylenrest,
R⁴, R⁵, R⁶ und R⁷ unabhängig voneinander bedeuten
a) Wasserstoff,
b) Halogen,
c) S(O)ₙR⁸,
d) OR⁸,
e) COOR⁸,
f) COR⁸,
g) CONR⁸R¹³,
h) CSNR⁸R¹³,
i) C(NR⁸)NR⁹R¹³,
j) NR¹⁴R¹⁵,
k) C₁₋₆-Alkyl, das gegebenenfalls substituiert ist mit Halogen, OR⁸, SR⁸, NR¹⁴R¹⁵, Phenyl, 5-6-gliedrigem Heteroaryl mit 1-4 N-, S- oder O-Atomen oder C₃₋₇-Cycloalkyl,
I) C₃₋₇-Cycloalkyl,
m) C₂₋₆-Alkenyl, gegebenenfalls substituiert mit Phenyl oder Halogen,
n) C₂₋₆-Alkinyl, gegebenenfalls substituiert mit Phenyl oder Halogen,
o) C₆₋₁₀-Aryl, das gegebenenfalls substituiert ist mit Halogen, CN, C₁₋₄-Alkyl, SR⁸ oder OR⁸,
p) 5-6-gliedriges Hetaryl mit 1 bis 4 N-, O- oder S-Atomen, das einen ankondensierten Benzolring enhalten und substituiert sein kann mit Halogen, NO₂, Cyano, -OR⁸, SR⁸, C₁₋₄-Alkyl, CF₃ oder NR⁸R¹³
q) CN,
r) NO₂,
s) CF₃,
t) OCF₃,
R⁴ und R⁵, R⁵ und R⁶ oder R⁶ und R⁷ bilden gemeinsam mit 2 benachbarten Kohlenstoffatomen eine 5-oder 6-gliedrigen Karbocyclus, der mit NR¹⁴R¹⁵ substituiert sein kann,
R⁸, R⁹ und R¹⁰ unabhängig voneinander bedeuten:
a) Wasserstoff,
b) C₁₋₆-Alkyl,
c) C₆₋₁₀-Aryl, das gegebenenfalls substituiert ist mit Halogen oder C₁₋₄-Alkyl
R¹³ bedeutet:
a) Wasserstoff,
b) C₁₋₆-Alkyl, gegebenenfalls substituiert mit Halogenen, Amino-, Hydroxyloder Sulfhydrylgruppen,
c) C₆₋₁₀-Aryl,
R14 und R¹⁵ bedeuten unabhängig voneinander:
d) Wasserstoff
e) CO₂R¹⁰
f) C₁₋₆-Alkyl, gegebenfalls substituiert mit Halogen, Hydroxy, C₁₋₄-Alkoxy, Nitro, Amino, C₁₋₆-Alkyl, Trifluormethyl, Carboxyl, Cyano, Carboxamido, C₃₋₇-Cycloalkyl, Indanyl, 1,2,3,4-Tetrahydronaphthyl, C₆₋₁₀-Aryl, 5- oder 6-gliedrigen Heteroaryl mit 1 - 4 Stickstoff-, Sauerstoffoder Schwefelatomen, wobei der Aryl- und der Heteroarylrest mit Halogen, Hydroxy, C₁₋₄-Alkoxy, C₁₋₄-Alkyl, CF₃, NO₂, NH₂, N(C₁₋₄-Alkyl)₂ oder Carboxyl substituiert sein können,
oder
R¹⁴ und R¹⁵ bilden gemeinsam mit dem Stickstoffatom einen 5 - 7-gliedrigen gesättigten Heterocyclus, der ein weiteres Sauerstoff-, Stickstoff- oder Schwefelatom enthalten und mit C₁₋₄-Alkyl, Phenyl, Benzyl oder Benzoyl substituiert sein kann oder einen ungesättigten 5-gliedrigen Heterocyclus, der 1 - 3 N-Atome enthalten und substituiert sein kann mit Phenyl, C₁₋₄, Alkyl Halogen oder CH₂-OH,
und
n bedeutet 0, 1 oder 2.

2. Verbindungen nach Anspruch 1, worin R⁴, R⁵, R⁶ und R⁷ bedeuten:
a) Wasserstoff
b) Halogen,
c) SR⁸,
d) OR⁸,
e) COOR⁸,
f) COR⁸,
g) CONR⁸R¹³,
h) NR¹⁴R¹⁵,
i) C₁₋₆-Alkyl, das gegebenenfalls substituiert ist mit Halogen, OR⁸, SR⁸, NR¹⁴R¹⁵, Phenyl, 5 - 6gliedrigem Heteroaryl mit 1 - 4 N-, S- oder O-Atomen oder C₃₋₇-Cycloalkyl,
j) Phenyl, das gegebenenfalls substituiert ist mit Halogen, CN, C₁₋₄-Alkyl, SR⁸ oder OR⁸,
k) 5 - 6gliedriges Heteroaryl mit 1 bis 4 N-, O- oder S-Atomen, das einen ankondensierten Benzolring enthalten und substituiert sein kann mit Halogen, NO₂, Cyano, C₁₋₄-Alkyl, CF₃ oder NR⁸R¹³,
l) CN,
m) NO₂,
n) CF₃,
o) OCF₃ oder
p) R⁴ und R⁵, R⁵ und R⁶ oder R⁶ und R⁷ bilden gemeinsam mit 2 benachbarten Kohlenstoffatomen einen 5- oder 6gliedrigen Karbocyclus, der mit NR¹⁴R¹⁵ substituiert sein kann.

3. Verbindungen nach Anspruch 1, worin R¹⁴ und R¹⁵ bedeuten:
a) Wasserstoff,
b) C₁₋₆-Alkyl, gegebenenfalls substituiert mit Halogen, Hydroxy, C₁₋₄-Alkoxy, Nitro, Amino, C₁₋₆-Alkyl, Trifluormethyl, Carboxyl, Cyano, Carboxamido, Phenyl, 5- oder 6gliedrigen Heteroaryl mit 1 - 4 Stickstoff-, Sauerstoffoder Schwefelatomen, wobei der Phenyl- und der Heteroarylrest mit Halogen, Hydroxy, C₁₋₄-Alkoxy, C₁₋₄-Alkyl, CF₃, NO₂, NH₂, N(C₁₋₄-Alkyl)₂ oder Carboxyl substituiert sein können.

4. 4-Amino-3a-fluor-2,3,3a,9b-tetrahydro-1H-cyclopenta[c]chinolin,
4-Amino-3a,6-difluor-2,3,3a,9b-tetrahydro-1H-cyclopenta[c]chinolin,
4-Amino-8-brom-3a-fluor-2,3,3a,9b-tetrahydro-1H-cyclopenta[c]chinolin,
4-Amino-8-chlor-3a-fluar-2,3,3a,9b-tetrahydro-1H-cyclopenta[c]chinolin,
4-Amino-7-methylaminomethyl-3a-fluor-2,3,3a,5,9b- tetrahydro-1Hcyclopenta[c]chinolin Dihydrochlorid,
4-Amino-7-(2-(methylamino)ethyl]-3a-fluor-2,3,3a,5,9b- tetrahydro-1Hcyclopenta[c]chinolin Dihydrochlorid,
4-Amino-7-[3-(3-chlorbenzylamino)propyl]-3a-fluor-2,3,3a,9b-tetrahydro-1H-cyclopenta[c]chinolin Dihydrochlorid, gemäß Anspruch 1.

5. Arzneimittel enthaltend eine Verbindung nach Anspruch 1 und übliche Träger- und Hilfsstoffe.

6. Verfahren zur Herstellung von Verbindungen nach Anspruch 1 **dadurch gekennzeichnet, daß** man eine Verbindung der Formel (II) oder deren Salz 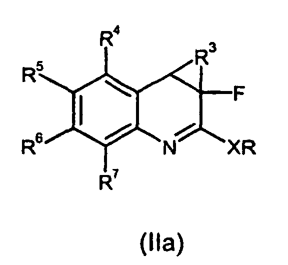 oder 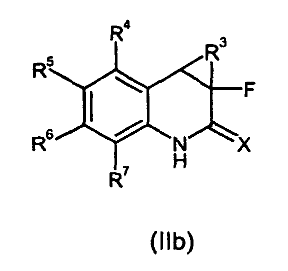 worin R³ bis R⁷ die obige Bedeutung haben, R Methyl oder Ethyl und X Sauerstoff oder Schwefel ist, mit Ammoniak, primären oder sekundären Aminen, Hydroxylamin und seinen Derivaten oder Hydrazin und seinen Derivaten umsetzt und gewünschtenfalls anschließend die Isomeren trennt oder die Salze bildet.

7. Verwendung der Verbindungen der Formel I nach Anspruch 1 zur Herstellung eines Arzneimittels zur Behandlung von Erkrankungen, die durch NOS-Synthasen ausgelöst werden.

8. Verwendung nach Anspruch 7 zur Behandlung neurodegenerativer Erkrankungen.

9. Verbindungen der Formel IIb) 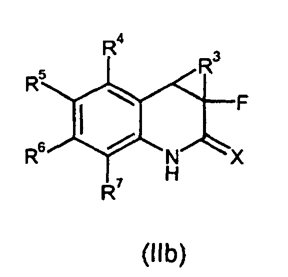 worin
R³ bis R⁷ die Bedeutung nach Anspruch 1 haben und X Sauerstoff oder Schwefel ist.

## Claims

1. Compounds of formula I, the tautomeric and isomeric forms thereof or salts thereof, 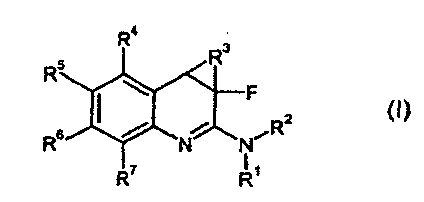 in which
R¹ and R² mean
hydrogen,
R³ means:
a C₁₋₅ alkylene radical,
R⁴, R⁵, R⁶ and R⁷, independently of one another, mean:
a) hydrogen,
b) halogen,
c) S(O)ₙR⁸,
d) OR⁸,
e) COOR⁸,
f) COR⁸,
g) CONR⁸R¹³,
h) CSNR⁸R¹³,
i) C(NR⁸)NR⁹R¹³,
j) NR¹⁴R¹⁵,
k) C₁₋₆ alkyl, which optionally is substituted with halogen, OR⁸, SR⁸, NR¹⁴R¹⁵, phenyl, 5- to 6-membered heteroaryl with 1-4 N, S or O atoms or, C₃₋₇ cycloalkyl,
l) C₃₋₇ cycloalkyl,
m) C₂₋₆ alkenyl, optionally substituted with phenyl or halogen,
n) C₂₋₆ alkynyl, optionally substituted with phenyl or halogen,
o) C₆₋₁₀ aryl, which optionally is substituted with halogen, CN, C₁₋₄ alkyl, SR⁸ or OR⁸,
p) 5- to 6-membered hetaryl with 1 to 4 N, O or S atoms, which may contain a benzene ring condensed thereto, and which can be substituted with halogen, NO₂, cyano, -OR⁸, SR⁸, C₁₋₄ alkyl, CF₃ or NR⁸R¹³,
q) CN,
r) NO₂,
s) CF₃,
t) OCF₃,
R⁴ and R⁵, R⁵ and R⁶, or R⁶ and R⁷ together with 2 adjacent carbon atoms form a 5- or 6-membered carbocycle, which can be substituted with NR¹⁴R¹⁵,
R⁸, R⁹ and R¹⁰, independently of one another, mean:
a) hydrogen,
b) C₁₋₆ alkyl, or
c) C₆₋₁₀ aryl, which optionally is substituted with halogen or C₁₋₄ alkyl,
R¹³ means:
a) hydrogen,
b) C₁₋₆ alkyl, optionally substituted with halogens, amino, hydroxyl or sulfhydryl groups, or
c) C₆₋₁₀ aryl,
R¹⁴ and R¹⁵, independently of one another, mean:
d) hydrogen
e) CO₂R¹⁰
f) C₁₋₆ alkyl, optionally substituted with halogen, hydroxy, C₁₋₄ alkoxy, nitro, amino, C₁₋₆ alkyl, trifluoromethyl, carboxyl, cyano, carboxamido, C₃₋₇ cycloalkyl, indanyl, 1,2,3,4-tetrahydronaphthyl, C₆₋₁₀ aryl, 5- or 6-membered heteroaryl with 1-4 nitrogen, oxygen or sulphur atoms, whereby the aryl and the heteroaryl radical may be substituted with halogen, hydroxy, C₁₋₄ alkoxy, C₁₋₄ alkyl, CF₃, NO₂, NH₂, N(C₁₋₄ alkyl)₂ or carboxyl, or
R¹⁴ and R¹⁵ together with the nitrogen atom form a 5- to 7-membered saturated heterocycle, which may contain another oxygen, nitrogen or sulphur atom and may be substituted with C₁₋₄ alkyl, phenyl, benzyl or benzoyl, or an unsaturated 5-membered heterocycle, which may contain 1-3 N atoms and can be substituted with phenyl, C₁₋₄ alkyl, halogen or CH₂-OH,
and
n means 0, 1 or 2.

2. Compounds according to Claim 1, in which R⁴, R⁵, R⁶ and R⁷ mean:
a) hydrogen,
b) halogen,
c) SR⁸,
d) OR⁸,
e) COOR⁸,
f) COR⁸,
g) CONR⁸R¹³,
h) NR¹⁴R¹⁵,
i) C₁₋₆ alkyl, which optionally is substituted with halogen, OR⁸, SR⁸, NR¹⁴R¹⁵, phenyl, 5- to 6-membered heteroaryl with 1-4 N, S or O atoms, or C₃₋₇ cycloalkyl,
j) phenyl, which optionally is substituted with halogen, CN, C₁₋₄ alkyl, SR⁸ or OR⁸,
k) 5- to 6-membered heteroaryl with 1 to 4 N, O or S atoms, which may contain a benzene ring condensed thereto, and which can be substituted with halogen, NO₂, cyano, C₁₋₄ alkyl, CF₃ or NR⁸R¹³,
l) CN,
m) NO₂,
n) CF₃,
o) OCF₃, or
p) R⁴ and R⁵, R⁵ and R⁶, or R⁶ and R⁷ together with 2 adjacent carbon atoms form a 5- or 6-membered carbocyclic compound, which can be substituted with NR¹⁴R¹⁵.

3. Compounds according to Claim 1, in which R¹⁴ and R¹⁵ mean
a) hydrogen,
b) C₁-C₆ alkyl, optionally substituted with halogen, hydroxy, C₁-C₄ alkoxy, nitro, amino, C₁-C₆ alkyl, trifluoromethyl, carboxyl, cyano, carboxamido, phenyl, 5- or 6-membered heteroaryl with 1 - 4 nitrogen, oxygen or sulphur atoms, whereby the phenyl and heteroaryl radical can be substituted with halogen, hydroxy, C₁₋₄ alkoxy, C₁₋₄ alkyl, CF₃, NO₂, NH₂, N(C₁₋₄ alkyl)₂ or carboxyl.

4. 4-Amino-3a-fluoro-2,3,3a,9b-tetrahydro-1H-cyclopenta[c]quinoline,
4-amino-3a,6-difluoro-2,3,3a,9b-tetrahydro-1H-cyclopenta[c]quinoline,
4-amino-8-bromo-3a-fluoro-2,3,3a,9b-tetrahydro-1H-cyclopenta[c]quinoline
4-amino-8-chloro-3a-fluoro-2,3,3a,9b-tetrahydro-1H-cyclopenta[c]quinoline,
4-amino-7-methylaminomethyl-3a-fluoro-2,3,3a,5,9b-tetrahydro-1H-cyclopenta[c]quinoline dihydrochloride,
4-amino-7-[2-(methylamino)ethyl]-3a-fluoro-2,3,3a,5,9b-tetrahydro-1H-cyclopenta[c]quinoline dihydrochloride,
4-amino-7-[3-(3-chlorobenzylamino)propyl]-3a-fluoro-2,3,3a,9b-tetrahydro-1H-cyclopenta[c]quinoline dihydrochloride, according to Claim 1.

5. Pharmaceutical composition comprising a compound according to Claim 1 and customary carriers and auxiliary substances.

6. Process for the production of compounds according to Claim 1, **characterized in that** a compound of formula (II) or its salt 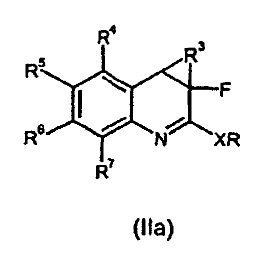 or 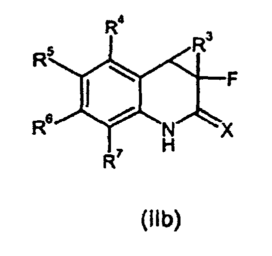 in which R³ to R⁷ have the above meaning, R is methyl or ethyl and X is oxygen or sulphur, is reacted with ammonia, primary or secondary amines, hydroxylamine and its derivatives or hydrazine and its derivatives, and, if desired, then the isomers are separated or the salts are formed.

7. Use of the compounds of formula (I) according to Claim 1 for the preparation of a pharmaceutical composition for the treatment of diseases which are triggered by NOS synthases.

8. Use according to Claim 7 for the treatment of neurodegenerative diseases.

9. Compounds of formula (IIb) 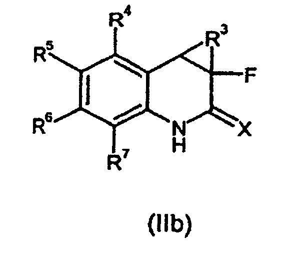 in which
R³ to R⁷ have the meaning according to Claim 1 and X is oxygen or sulphur.

## Revendications

1. Composés de formule I, leurs formes tautomères et isomères ou sels 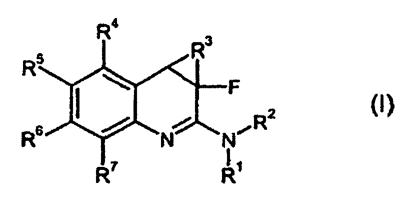 formule dans laquelle
R¹ et R² représentent :
un atome d'hydrogène,
R³ représente :
un radical alkylène en C₁-C₅,
R⁴, R⁵, R⁶ et R⁷ représentent, indépendamment les uns des autres
a) un atome d'hydrogène,
b) un atome d'halogène,
c) S(O)ₙR⁸,
d) OR⁸,
e) COOR⁸,
f) COR⁸,
g) CONR⁸R¹³,
h) CSNR⁸R¹³,
i) C(NR⁸)NR⁹R¹³,
j) NR¹⁴R¹⁵,
k) un groupe alkyle en C₁-C₆ qui est éventuellement substitué par halogène, OR⁸, SR⁸, NR¹⁴R¹⁵, phényle, hétéroaryle à 5-6 chaînons comportant 1-4 atomes de N, S ou O ou cycloalkyle en C₃-C₇,
l) un groupe cycloalkyle en C₃-C₇,
m) un groupe alcényle en C₂-C₆, éventuellement substitué par phényle ou halogène,
n) un groupe alcynyle en C₂-C₆, éventuellement substitué par phényle ou halogène,
o) un groupe aryle en C₆-C₁₀ qui est éventuellement substitué par halogène, CN, alkyle en C₁-C₄, SR⁸ou OR⁸,
p) un groupe hétéroaryle à 5-6 chaînons comportant de 1 à 4 atomes de N, O ou S, qui peut comporter un noyau benzénique fusionné et peut être substitué par halogène, NO₂, cyano, -OR⁸, SR⁸, alkyle en C₁-C₄, CF₃ ou NR⁸R¹³,
q) CN,
r) NO₂,
s) CF₃,
t) OCF₃,
R⁴ et R⁵, R⁵ et R⁶ ou R⁶ et R⁷ forment ensemble avec deux atomes de carbone voisins un cycle carbocyclique à 5 ou 6 chaînons, qui peut être substitué par NR¹⁴R¹⁵,
R⁸, R⁹ et R¹⁰ représentent, indépendamment les uns des autres :
a) un atome d'hydrogène,
b) un groupe alkyle en C₁-C₆,
c) un groupe aryle en C₆-C₁₀ qui est éventuellement substitué par halogène ou alkyle en C₁-C₄,
R¹³ représente :
a) un atome d'halogène,
b) un groupe alkyle en C₁-C₆ qui est éventuellement substitué par des atomes d'halogène, des groupes amino, hydroxy ou sulfhydryle,
c) un groupe aryle en C₆-C₁₀,
R¹⁴ et R¹⁵ représentent indépendamment l'un de l'autre :
d) un atome d'hydrogène,
e) CO₂R¹⁰,
f) un groupe alkyle en C₁-C₆ qui est éventuellement substitué par halogène, hydroxy, alcoxy en C₁-C₄, nitro, amino, alkyle en C₁-C₆, trifluorométhyle, carboxy, cyano, carboxamido, cycloalkyle en C₃-C₇, indanyle, 1,2,3,4-tétrahydronaphtyle,
un radical aryle en C₆-C₁₀, hétéroaryle à 5 ou 6 chaînons ayant de 1 à 4 atomes d'azote, d'oxygène ou de soufre, le radical aryle et le radical hétéroaryle pouvant être substitués par halogène, hydroxy, alcoxy en C₁-C₄, alkyle en C₁-C₄, CF₃, NO₂, NH₂, N[alkyle(C₁-C₄)]₂ ou carboxy, ou
R¹⁴ et R¹⁵ forment ensemble, avec l'atome d'azote, un hétérocycle saturé à 5-7 chaînons qui peut contenir un autre atome d'oxygène, d'azote ou de soufre et être substitué par alkyle en C₁-C₄, phényle, benzyle ou benzoyle, ou un hétérocycle insaturé à 5 chaînons qui peut contenir 1-3 atomes et être substitué par un atome d'halogène ou par un groupe phényle, alkyle en C₁-C₄ ou CH₂OH,
et
n représente 0, 1 ou 2.

2. Composés selon la revendication 1, dans lesquels R⁴, R⁵, R⁶ et R⁷ représentent :
a) un atome d'hydrogène,
b) un atome d'halogène,
c) SR⁸,
d) OR⁸,
e) COOR⁸,
f) COR⁸,
g) CONR⁸R¹³,
h) NR¹⁴R¹⁵,
i) un groupe alkyle en C₁-C₆ qui est éventuellement substitué par halogène, OR⁸, SR⁸, NR¹⁴R¹⁵, phényle, hétéroaryle à 5-6 chaînons comportant 1-4 atomes de N, S ou O ou cycloalkyle en C₃-C₇,
j) un groupe phényle qui est éventuellement substitué par halogène, CN, alkyle en C₁-C₄, SR⁸ ou OR⁸,
k) un groupe hétéroaryle à 5-6 chaînons comportant de 1 à 4 atomes de N, O ou S, qui peut comporter un noyau benzénique fusionné et peut être substitué par halogène, NO₂, cyano, alkyle en C₁-C₄, CF₃ ou NR⁸R¹³,
l) CN,
m) NO₂,
n) CF₃,
o) OCF₃ ou
p) R⁴ et R⁵, R⁵ et R⁶ ou R⁶ et R⁷ forment ensemble avec deux atomes de carbone voisins un cycle carbocyclique à 5 ou 6 chaînons, qui peut être substitué par NR¹⁴R¹⁵,

3. Composés selon la revendication 1, dans lesquels R¹⁴ et R¹⁵ représentent :
a) un atome d'hydrogène,
b) un groupe alkyle en C₁-C₆ qui est éventuellement substitué par halogène, hydroxy, alcoxy en C₁-C₄, nitro, amino, alkyle en C₁-C₆, trifluorométhyle, carboxy, cyano, carboxamido, un radical phényle, hétéroaryle à 5 ou 6 chaînons ayant de 1 à 4 atomes d'azote, d'oxygène ou de soufre, le radical phényle et le radical hétéroaryle pouvant être substitués par halogène, hydroxy, alcoxy en C₁₋₄, alkyle en C₁₋₄, CF₃, NO₂, NH₂, N[alkyle(C₁-C₄)]₂ ou carboxy.

4. 4-amino-3a-fluoro-2,3,3a,9b-tétrahydro-1H-cyclopenta(c)quinoléine,
4-amino-3a,6-difluoro-2,3,3a,9b-tétrahydro-1H-cyclopenta(c)quinoléine,
4-amino-8-bromo-3a-fluoro-2,3,3a,9b-tétrahydro-1H-cyclopenta(c)quinoléine,
4-amino-8-chloro-3a-fluoro-2,3,3a,9b-tétrahydro-1H-cyclopenta(c)quinoléine, dichlorhydrate de 4-amino-7-méthylaminométhyle-3a-fluoro-2,3,3a,5,9b-tétrahydro-1H-cyclopenta(c)quinoléine,
dichlorhydrate de 4-amino-7-[2-(méthylamino)éthyl]-3a-fluoro-2,3,3a,5,9b-tétrahydro-1H-cyclopenta(c)quinoléine,
dichlorhydrate de 4-amino-7-[3-(3-chlorobenzylamino)propyl]-3a-fluoro-2,3,3a,9b-tétrahydro-1H-cyclopenta(c)quinoléine,
selon la revendication 1.

5. Médicament contenant un composé selon la revendication 1 et des véhicules et adjuvants usuels.

6. Procédé pour la préparation de composés selon la revendication 1, **caractérisé en ce qu'**on fait réagir un composé de formule (II) ou un sel d'un tel composé 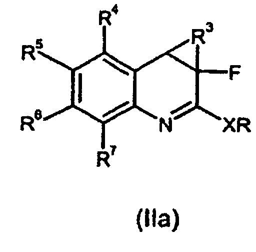 ou 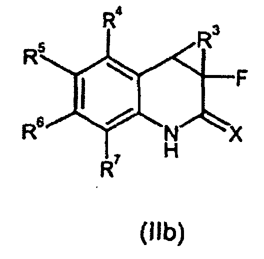 formules dans lesquelles R³ à R⁷ ont les significations données plus haut, R représente le groupe méthyle ou éthyle et X est un atome d'oxygène ou de soufre, avec de l'ammoniac, des amines primaires ou secondaires, de l'hydroxylamine et ses dérivés ou de l'hydrazine et ses dérivés, et, si on le désire, ensuite on sépare les isomères ou on forme les sels.

7. Utilisation des composés de formule I selon la revendication 1, pour la fabrication d'un médicament destiné au traitement de maladies qui sont déclenchées par des NOS (monoxyde d'azote synthases).

8. Utilisation selon la revendication 7, pour le traitement de maladies neurodégénératives.

9. Composés de formule IIb 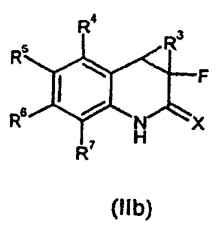 dans laquelle
R³ à R⁷ ont les significations selon la revendication 1 et X est un atome d'oxygène ou de soufre.
